(19) Europäisches Patentamt
European Patent Office
Office européen des brevets

(11) Veröffentlichungsnummer : **0 457 714 A1**

# (12) EUROPÄISCHE PATENTANMELDUNG

(21) Anmeldenummer : **91810168.4**

(22) Anmeldetag : **13.03.91**

(51) Int. Cl.[5] : **C07D 513/04**, C07D 237/04, C07C 331/28, A01N 43/90, // (C07D513/04, 285:00, 237:00)

(30) Priorität : **22.03.90 CH 951/90**
**22.03.90 CH 949/90**

(43) Veröffentlichungstag der Anmeldung :
**21.11.91 Patentblatt 91/47**

(84) Benannte Vertragsstaaten :
**AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Pissiotas, Georg, Dr.**
**Am Sonnenrain 71**
**W-7850 Lörrach (DE)**
Erfinder : **Moser, Hans, Dr.**
**Hauptstrasse 67B**
**CH-4312 Magden (CH)**
Erfinder : **Brunner, Hans-Georg, Dr.**
**Wannenstrasse 14**
**CH-4415 Lausen (CH)**

(54) **Thiadiazabicyclononanderivate, Verfahren zu ihrer Herstellung, Zwischenprodukte und ihre Verwendung als Herbizide.**

(57) Verbindungen der Formel I

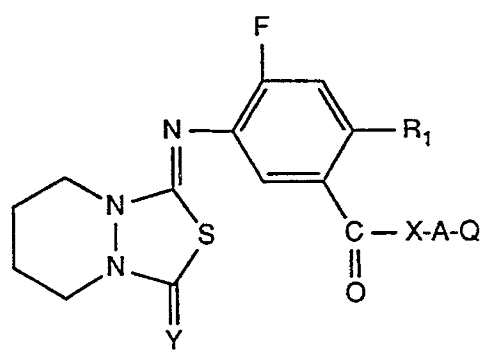

(I), worin

$R_1$ Halogen ;
X Sauerstoff ; oder Schwefel ;
Y Sauerstoff ; oder Schwefel ;
A eine geradkettige oder verzweigte $C_1$-$C_4$-Alkylenkette ;
Q Hydroxyl ; Halogen ; Cyano ; unsubstituiertes oder durch Cyano oder Halogen substituiertes $C_2$-$C_6$-Alkenyl ; $C_2$-$C_4$-Alkinyl ; $-CR_2{=}CH\text{-}COOR_3$ ; $-CH[N(R_2)_2]COOR_2$ ; $-NR_4(R_5)$ ; $-CO\text{-}NR_6R_7$ ; $-COON{=}CR_8(R_8)$ ; $-C(R_2)(OR_9)_2$ ; $-Si(R_{10})_3$ ; $-COOCH_2Si(CH_3)_2\text{-}C_1\text{-}C_6$-Alkyl ;

$-\underset{\underset{O}{\|}}{P}(OR_{11})O_mR_{11}$ ;

$-CON(R_{12})SO_2\text{-}C_1\text{-}C_6$-Alkyl ; $-CON(R_{12})SO_2\text{-}C_1\text{-}C_4$-Halogenalkyl ; $C_1$-$C_6$-Alkylcarbonyl ; $C_2$-$C_6$-Alkoxyalkylcarbonyl ; Benzoyl ; Benzylcarbonyl ; $-COOR_{16}$ ; $-CO\text{-}N(R_2)CH_2\text{-}CH(O\text{-}C_1\text{-}C_6\text{-Alkyl})_2$ ; $-COO(CH_2)_jN(R_2)_2$ ; $-S(O)_k\text{-}R_{14}$ ; $-S(O)_k\text{-}A'\text{-}COOR_{13}$ ; oder
einen über Kohlenstoff oder Stickstoff gebundenen 5- oder 6-gliedrigen Heterocyclus, welcher 1 bis 3 Heteroatome ausgewählt aus der Gruppe N, O und S enthält, wobei diese Heterocyclen ihrerseits noch benzanelliert sein und bis zu zweifach durch Halogen, $C_1$-$C_4$-Alkyl, $C_1$-$C_3$-Halogenalkyl, $C_1$-$C_3$-Alkoxy, $C_1$-$C_3$-Halogenalkoxy, $C_1$-$C_3$-Dialkylamino, Hydroxy oder Carbonylgruppen substituiert sein können ;
bedeutet,
haben gute pre- und postemergent-selektive herbizide und wuchsregulierende Eigenschaften.

EP 0 457 714 A1

## NEUE HERBIZIDE

Die vorliegende Erfindung betrifft neue, herbizid wirksame und pflanzenwuchsregulierende Thiadiazabicyclononane, Verfahren zu ihrer Herstellung, neue Zwischenprodukte, die sie als Wirkstoffe enthaltende Mittel sowie deren Verwendung zum Bekämpfen von Unkräutern, vor allem selektiv in Nutzpflanzenkulturen oder zum Regulieren und Hemmen des Pflanzenwachstums.

Thiadiazabicyclononane mit herbizider Wirkung sind aus den Europäischen Patentanmeldungen EP-A-0 238 711 und EP-A-0 273 417 bekanntDie dort offenbarten Wirkstoffe können jedoch die Anforderungen bezüglich Wirkungsstärke und Selektivität nicht immer erfüllen. Es besteht somit ein Bedarf nach besser wirksamen und selektiveren Wirkstoffen.

Es wurden nun neue Thiadiazabicyclononane mit verbesserter herbizider und pflanzenwuchsregulierender Wirkung gefunden.

Die erfindungsgemässen Thiadiazabicyclononane entsprechen der Formel I

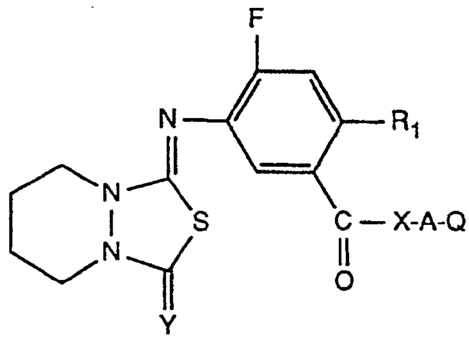

(I)

worin

$R_1$ Halogen;

X Sauerstoff; oder Schwefel;

Y Sauerstoff; oder Schwefel;

A eine geradkettige oder verzweigte $C_1$-$C_4$-Alkylenkette;

Q Hydroxyl; Halogen; Cyano; unsubstituiertes oder durch Cyano oder Halogen substituiertes $C_2$-$C_6$-Alkenyl; $C_2$-$C_4$-Alkinyl; $-CR_2{=}CH-COOR_3$; $-CH[N(R_2)_2]COOR_2$; $-NR_4(R_5)$; $-CO-NR_6R_7$; $-COON{=}CR_8(R_8)$; $-C(R_2)(OR_9)_2$; $-Si(R_{10})_3$; $-COOCH_2Si(CH_3)_2$-$C_1$-$C_6$-Alkyl;

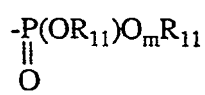 ;

$-CON(R_{12})SO_2$-$C_1$-$C_6$-Alkyl; $-CON(R_{12})SO_2$-$C_1$-$C_4$-Halogenalkyl; $C_1$-$C_6$-Alkylcarbonyl; $C_2$-$C_8$-Alkoxyalkylcarbonyl; Benzoyl; Benzylcarbonyl; $-COOR_{16}$; $-CO-N(R_2)CH_2-CH(O$-$C_1$-$C_6$-Alkyl$)_2$; $-COO(CH_2)_jN(R_2)_2$; $-S(O)_k-R_{14}$; $-S(O)_k-A'-COOR_{13}$; oder

einen über Kohlenstoff oder Stickstoff gebundenen 5- oder 6-gliedrigen Heterocyclus, welcher 1 bis 3 Heteroatome ausgewählt aus der Gruppe N, O und S enthält, wobei diese Heterocyclen ihrerseits noch benzanelliert sein und bis zu zweifach durch Halogen, $C_1$-$C_4$-Alkyl, $C_1$-$C_3$-Halogenalkyl, $C_1$-$C_3$-Alkoxy, $C_1$-$C_3$-Halogenalkoxy, $C_1$-$C_3$-Dialkylmino, Hydroxy oder Carbonylgruppen substituiert sein können;

$R_2$ Wasserstoff; $C_1$-$C_6$-alkyl; oder $C_2$-$C_6$Alkoxyalkyl;

$R_3$ $C_1$-$C_6$-Alkyl; oder $C_1$-$C_6$-Hydroxyalkyl;

$R_4$ und $R_5$ unabhängig voneinander je Wasserstoff; $C_1$-$C_6$-Alkyl; $C_2$-$C_6$Alkoxyalkyl; $C_3$-$C_6$-Alkenyl; $C_3$-$C_6$-Alkinyl; $C_3$-$C_6$-Cycloalkyl; 2-Furanylmethyl; 2-Tetrahydrofuranylmethyl; 2-(5-Methyl)-tetrahydrofuranylmethyl; oder 2-Thienylmethyl;

$R_6$ und $R_7$ unabhängig voneinander je Wasserstoff; $C_1$-$C_{12}$-Alkyl; $C_3$-$C_8$-Alkenyl; $C_3$-$C_6$-Alkinyl; $C_2$-$C_8$Alkoxyalkyl; $C_1$-$C_4$-Alkoxy; Benzyl; Phenyl; oder Cyano-$C_1$-$C_4$-alkyl; oder

$R_6$ und $R_7$ zusammen mit dem Stickstoffatom, an das sie gebunden sind, einen unsubstituierten oder bis zu zweifach durch $C_1$-$C_4$-Alkyl substituierten Pyrrolidino-, Piperidino-, Morpholino-, Thiomorpholino-, 4-Methylpiperazino-, Pyrazolino-, Imidazolino- oder 1,2,4-Triazolinrest;

$R_8$ unabhängig voneinander je $C_1$-$C_6$-Alkyl; oder zusammen eine $C_3$-$C_7$-Alkylenkette;

$R_9$ unabhängig voneinander $C_1$-$C_4$-Alkyl; oder $C_1$-$C_4$-Haloalkyl; oder zusammen eine Ethano-, Propano- oder Cyclohexan-1,2-diylbrücke;

$R_{10}$ unabhängig voneinander $C_1$-$C_6$-Alkyl; oder $C_1$-$C_6$-Alkoxy;

$R_{11}$ unabhängig voneinander Wasserstoff; $C_1$-$C_6$-Alkyl; $C_1$-$C_6$-Haloalkyl; Cyano-$C_1$-$C_6$-alkyl; $C_3$-$C_4$-alkenyl; oder $C_3$-$C_4$-Alkinyl

$R_{12}$ $C_1$-$C_4$-Alkyl; oder $C_3$-$C_7$-Cycloalkyl;

A' eine geradkettige oder verzweigte $C_1$-$C_4$-Alkylenkette;

$R_{13}$ Wasserstoff; $C_1$-$C_{12}$-Alkyl; $C_3$-$C_7$-Alkenyl; $C_3$-$C_6$-Alkinyl; $C_3$-$C_7$-Cycloalkyl; $C_2$-$C_8$-Alkoxyalkyl; $C_3$-$C_5$-Alkenyloxy-$C_1$-$C_4$-alkyl;$C_1$-$C_4$-Thioalkyl-$C_1$-$C_4$-alkyl; oder $C_1$-$C_4$-Dialkylamino-$C_1$-$C_4$-alkyl;

$R_{14}$ $C_1$-$C_{10}$-Alkyl;

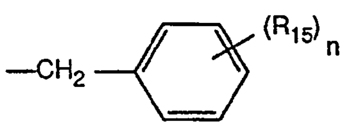 ; oder 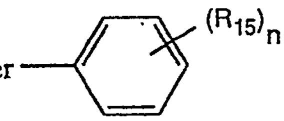 ;

$R_{15}$ $C_1$-$C_4$-Alkyl; Halogen; oder $C_1$-$C_4$-Alkoxy;

$R_{16}$ Wasserstoff; $C_3$-$C_7$-Alkenyl; $C_3$-$C_6$-Alkinyl; $C_3$-$C_7$-Cycloalkyl; $C_2$-$C_8$-Alkoxyalkyl; $C_3$-$C_5$-Alkenyloxy-$C_1$-$C_4$-alkyl; $C_2$-$C_8$-Alkylthioalkyl; $C_1$-$C_4$-Dialkylamino-$C_1$-$C_4$-alkyl; oder, wenn $R_1$ für Chlor und Y für Sauerstoff steht, $C_1$-$C_{10}$-Alkyl;

j 0; 2; oder 3;

k 0; 1; oder 2;

m 0; oder 1; und

n 0; 1; 2; oder 3;

bedeutet, mit der Massgabe, dass A nicht für Methano steht wenn X Sauerstoff und Q Hydroxyl oder Halogen bedeutet.

Die Verbindungen der Formel I können durch die Reste A und Q unsymmetrisch substituiert sein. Die Erfindung umfasst sowohl das Racemat als auch die angereicherten und optisch reinen Formen der jeweiligen Stereoisomeren.

Die unsymmetrisch substituierten Verbindungen der Formel I fallen, sofern nicht chirale Edukte verwendet werden, im allgemeinen bei den in dieser Anmeldung beschriebenen Verfahren als Racemate an. Die Stereoisomeren können sodann nach an sich bekannten Methoden, wie etwa fraktionierter Kristallisation nach Salzbildung mit optisch reinen Basen, Säuren oder Metallkoplexan, oder aber durch chromatographische Verfahren aufgrund physikochemischer Eigenschaften getrennt werden.

In den obigen Definitionen ist unter Halogen, Fluor, Chlor, Brom und Jod vorzugsweise Fluor, Chlor und Brom zu verstehen, insbesondere bei $R_1$ Chlor und Brom.

A als $C_1$-$C_4$-Alkylenkette ist Methano, Ethano (Ethan-1,2-diyl), 1-Methylethano, 2-Methylethano, Propano (Propan-1,3-diyl), 1-Methylpropano, 2-Methylpropano, 3-Methylpropano, Butano und Ethan-1,1-diyl. Bevorzugt sind neben Ethano die in Nachbarstellung zum Substituenten X verzweigten Alkylenketten, wie 1-Methylethano oder 1-Ethylethano, insbesondere aber Ethano und 1-Methylethano.

Q als -$S(O)_k$-$R_{14}$ ist ein Thioether (k = 0), ein $R_{14}$-Sulfinyl-Radikal (k = 1) oder ein $R_{14}$-Sulfonyl-Radikal (k = 2).

In den Fällen, in denen $R_{14}$ Alkyl bedeutet, sind die Thioether bevorzugt. Wenn $R_{14}$ für ein gebebenenfalls substituiertes Benzyl- oder Phenylradikal steht, sind neben den Thioethern die Sulfonylstrukturen (k = 2) bevorzugt.

Di-Alkylamino ist Dimethylamino, Methylethylamino, Diethylamino, Di-Butylamino und Di-Isopropylamino.

Q als Heterocyclus sind sowohl ungesättigte, wie auch ganz oder teilweise gesättigte Heterocyclen, insbesondere Pyridin-2-yl, 1,3-Thiazol-5-yl, Thiophen-2-yl, Pyrrolidin-2-on-1-yl, Pyrrolidin-1-yl, Morpholin-4-yl, Furan-2-yl, Indol-1-yl, Pyrrol-1-yl, Imidazol-1-yl, Pyrazol-1-yl und 1,2,4-Triazol-1-yl. Diese Heterocyclen können ihrerseits substituiert sein, wie etwa 1-Methylpyrazol-4-yl, 5-Methylfuran-2-yl, 4-Methyl-1,3-thiazol-5-yl oder Pyrrolidin-2-on-1-yl.

Alkyl ist Methyl, Ethyl, Isopropyl, n-Propyl, n-Butyl, iso-Butyl, sek-Butyl, tert-Butyl sowie die verschiedenen isomeren Pentyl-, Hexyl-, Heptyl-, Octyl-, Nonyl-, Decyl-, Undecyl- und Dodecylradikale. Bevorzugt ist Methyl, Ethyl, Isopropyl, n-Propyl und n-Butyl.

Halogenalkyl sind beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl; vorzugsweise Trichlormethyl, Difluorchlormethyl, Trifluormethyl und Dichlofluormethyl.

Alkoxy ist beispielsweise Methoxy, Ethoxy, Propyloxy, i-Propyloxy, n-Butyloxy, i-Butyloxy, s-Butyloxy und

t-Butyloxy; vorzugsweise Methoxy und Ethoxy.

Halogenalkoxy ist z.B. Fluormethoxy, Difluormethoxy, Trifluormethoxy, 2,2,2-Trifluorethoxy, 1,1,2,2-Tetrafluorethoxy, 2-Fluorethoxy, 2-Chlorethoxy und 2,2,2-Trichlorethoxy; vorzugsweise Difluormethoxy, 2-Chlorethoxy und Trifluormethoxy.

Alkylthio ist beispielsweise Methylthio, Ethylthio, Propylthio, Isopropylthio, n-Butylthio, i-Butylthio, s-Butylthio, t-Butylthio oder die isomeren Pentylthio, vorzugsweise Methylthio und Ethylthio.

$C_2$-$C_8$-Alkoxyalkyl oder $C_2$-$C_8$-Alkylthioalkyl sind vorzugsweise $C_1$-$C_4$-Alkoxy-$C_1$-$C_4$-alkyl oder $C_1$-$C_4$-Alkylthio-$C_1$-$C_4$-alkyl, während $C_2$-$C_6$-Alkoxyalkyl vorzugsweise $C_1$-$C_3$-Alkoxy-$C_1$-$C_3$-alkyl ist.

Unter Alkenyl ist geradkettiges oder verzweigtes Alkenyl zu verstehen, wie z.B. Vinyl, Allyl, Methallyl, 1-Methylvinyl, But-2-en-1-yl, Pentenyl, 2-Hexenyl oder 3-Heptenyl. Bevorzugt sind Alkenylreste mit einer Kettenlänge von 2 bis 3 Kohlenstoffatomen. Wenn der Alkenylrest an ein Heteroatom (Stickstoff, Sauerstoff oder Schwefel) gebunden und unsubstituiert ist, ist der Alkenylrest über ein gesättigtes Kohlenstoffatom gebunden.

Die in den Definitionen der Substituenten vorkommenden Alkinylreste können geradkettig oder verzweigt sein wie beispielsweise Ethinyl, Propargyl, 3-Butinyl, 1-Methylpropargyl, 1-Pentinyl oder 2-Hexinyl. Bevorzugt sind Ethinyl und Propargyl. Wenn der Alkinylrest an ein Heteroatom (Stickstoff, Sauerstoff oder Schwefel) gebunden und unsubstituiert ist, ist der Alkinylrest über ein gesättigtes Kohlenstoffatom gebunden.

Cycloalkyl steht beispielsweise für Cyclopropyl, Dimethylcyclopropyl, Cyclobutyl, Cyclopentyl, Methylcyclopentyl, Cyclohexyl oder Cycloheptyl, vorzugsweise aber für Cyclopropyl, Cyclopentyl oder Cyclohexyl.

In den Substituenten, welche aus mehreren Grundelementen zusammengesetzt sind, können die Teilelemente innerhalb der Definitionsbreite frei gewählt werden.

Bevorzugt sind Verbindungen der Formel I, worin

$R_1$ Fluor; Chlor; oder Brom;

A eine geradkettige oder verzweigte $C_1$-$C_4$-Alkylenkette;

n 0; 1; oder 2;

Q $C_1$-$C_{10}$-Alkylthio; $COOR_{16}$; $-S(O)_kR_{14}$; $-NR_4(R_5)$; einen gegebenenfalls bis zu zweifach durch $C_1$-$C_4$-Alkyl oder einfach durch eine Carbonylgruppe substituierten heterocyclischen Rest ausgewählt aus der Gruppe Pyridinyl, 1,3-Thiazolyl, Thiophenyl, Pyrrolidinyl, Morpholinyl, Furanyl, Indolyl, Pyrazolyl, 1,2-Oxazolyl, Pyrrolyl, Imidazolyl, Pyrazolyl und 1,2,4-Triazolyl;

$R_4$ und $R_5$ unabhängig voneinander $C_1$-$C_4$-Alkyl;

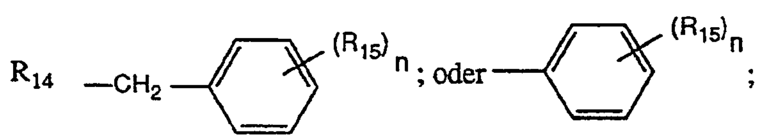

k 0; 1; oder 2;

n 0; 1; 2; oder 3;

$R_{15}$ $C_1$-$C_4$-Alkyl; Fluor, Chlor, Brom; oder $C_1$-$C_4$-Alkoxy; und

$R_{16}$ Cyclohexyl; $C_1$-$C_4$-Alkoxy-$C_1$-$C_4$-alkyl; $C_1$-$C_4$-Alkylthio-$C_1$-$C_4$-alkyl; $C_1$-$C_4$-Dialkylamino-$C_1$-$C_4$-alkyl; oder, wenn $R_1$ für Chlor und Y für Sauerstoff steht, $C_1$-$C_{10}$-Alkyl;

bedeutet.

Besonders bevorzugt sind Verbindungen der Formel I, worin

$R_1$ Chlor, oder Brom;

A Ethano; 1-Methylethano; 2-Methylethano; Methano;oder Propano;

Q $C_1$-$C_5$-Alkylthio; $COOR_{16}$; $-S(O)_kR_{14}$; Pyridin-2-yl; 1,3-Thiazol-5-yl; Thiophen-2-yl; Pyrrolidin-2-on-1-yl; Pyrrolidin-1-yl; Morpholin-4-yl; Furan-2-yl; Indol-1-yl; Pyrrol-1-yl; Imidazol-1-yl; Pyrazol-1-yl; 1,2,4-Triazol-1-yl; 4-Methyl-1,3-thiazol-5-yl; oder 5-Methylfuran-2-yl;

k 0; 1; oder 2;

$R_{14}$ Benzyl; oder Phenyl;

$R_{16}$ Cyclohexyl; $C_1$-$C_4$-Alkoxy-$C_1$-$C_4$-alkyl; $C_1$-$C_4$-Alkylthio-$C_1$-$C_4$-alkyl; $C_1$-$C_4$-Dialkylamino-$C_1$-$C_4$-alkyl; oder, wenn $R_1$ für Chlor und Y für Sauerstoff steht, $C_1$-$C_{10}$-Alkyl;

bedeutet.

Hervorzuheben sind aus dem Umfang der Verbindungen der Formel I sowie aus dem bevorzugt und besonders bevorzugten Bereich diejenigen Verbindungen der Formel I, worin

a) X Sauerstoff bedeutet;

b) Y Sauerstoff bedeutet;

c) A $-CH_2-CH_2-$ bedeutet;

d) A $-CH-CH_2-$ bedeutet;
$CH_3$

e) A $-CH_2-CH-$ bedeutet;
$CH_3$

f) Q $C_1$-$C_5$-Alkylthio bedeutet;
g) Q $-NR_4(R_5)$ und
$R_4$ und $R_5$ unabhängig voneinander $C_1$-$C_4$-Alkyl bedeuten;
h) $R_1$ Chlor oder Brom bedeutet;
i) $R_1$ Chlor bedeutet;
j) X Schwefel, Y Sauerstoff und Q $COOR_{16}$ bedeuten.
k)A $-CH_2-$ bedeutet;
l) A $-CH_2-CH_2-CH_2-$ bedeutet.
Weiterhin betrifft die Erfindung die Verbindungen der Formel Ia

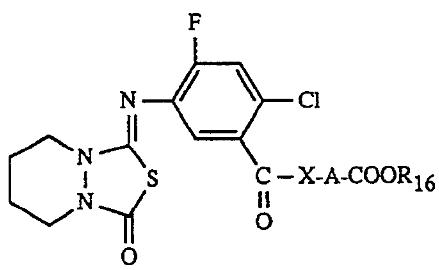

(Ia)

worin
X Sauerstoff oder Schwefel;
A eine geradkettige oder verzweigte $C_1$-$C_4$-Alkylenkette und
$R_{16}$ $C_1$-$C_{10}$-Alkyl bedeutet.
Besonders gut wirksam sind Verbindungen der Formel Ia, in denen A
m) $-CH_2-CH_2-$ bedeutet;

n) $-CH-CH_2-$ bedeutet;
$CH_3$

$CH_3$
o) $-CH-$ bedeutet;

p) $-CH_2-CH-$ bedeutet;
$CH_3$

q) $-CH_2-$ bedeutet; oder
r) $-CH(CH_3)-$ bedeutet.
Aus diesen Gruppen von Verbindungen der Formel Ia fallen insbesondere diejenigen ins Auge, bei denen
X Sauerstoff; oder Schwefel, insbesondere Schwefel;

$CH_3$
A $-CH-$ ; und

$R_{16}$ $C_1$-$C_6$-Alkyl
bedeutet.

Weiterhin sind aus der Gruppe der Verbindungen der Formel Ia diejenigen bevorzugt, bei denen

X Sauerstoff; oder Schwefel, insbesondere Sauerstoff;

A $CH_2$; und

$R_{16}$ $C_1$-$C_6$-Alkyl

bedeutet.

Namentlich zu nennen sind insbesondere die Verbindungen

9-[4-Chlor-2-fluor-5-(methoxycarbonylmethylthiocarbonyl)-phenylimino]-8-thia-1,6-diazabicyclo[4.3.0]nonan-7-on,

9-[4-Chlor-2-fluor-5-(1-methoxycarbonyl-ethylthiocarbonyl)-phenylimino]-8-thia-1,6-diazabicyclo[4.3.0]nonan-7-on,

9-[4-Chlor-2-fluor-5-(methoxycarbonylmethylthiocarbonyl)-phenylimino]-8-thia-1,6-diazabicyclo[4.3.0]nonan-7-on und

9-[4-Chlor-2-fluor-5-(methoxycarbonylmethoxycarbonyl)-phenylimino]-8-thia-1,6-diazabicyclo[4.3.0]nonan-7-on.

Weiterhin betrifft die Erfindung Verbindungen der Formel Ib,

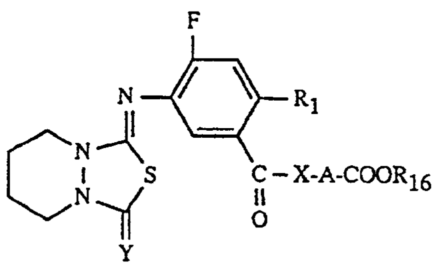
(Ib),

worin

Y Sauerstoff;

X Sauerstoff oder Schwefel;

A eine $C_1$-$C_2$-Alkylenkette; und

$R_{16}$ $C_1$-$C_4$-Alkoxy-$C_1$-$C_4$-alkyl; oder $C_1$-$C_4$-Alkylthio-$C_1$-$C_4$-alkyl; bedeutet.

Bei den Verbindungen der Formel Ib sind insbesondere diejenigen Untergruppen hervorzuheben, in denen

a) A $-CH_2-$;

b) A $-CH_2-CH_2-$; oder

c) A $-CH(CH_3)-$ bedeutet.

Als besonders bevorzugte Einzelverbindungen der Formel Ib sind zu nennen:

9-[4-Chlor-2-fluor-5-(2-methoxy-ethoxycarbonylmethylthiocarbonyl)-phenylimino]-8-thia 1,6-diazabicyclo[4.3.0]nonan-7-on (Verb. No. 1.49),

9-[4-Chlor-2-fluor-5-(2-methoxy-1-methyl-ethoxycarbonylmethylthiocarbonyl)-phenylimino]-8-thia 1,6-diazabicyclo[4.3.0]nonan-7-on (Verb. No. 1.51 ),

9-[4-Chlor-2-fluor-5-(2-methoxy-1-methyl-ethoxycarbonyl-1-ethylthiocarbonyl)-phenylimino]-8-thia1,6-diazabicyclo[4.3.0]nonan-7-on (Verb. No. 1.55),

9-[4-Chlor-2-fluor-5-(2-methoxy-ethoxycarbonyl-1-ethylthiocarbonyl)-phenylimino]-8-thia1,6-diazabicyclo[4.3.0]nonan-7-on (Verb. No. 1.54) und

9-[4-Chlor-2-fluor-5-(2-ethoxy-ethoxycarbonylmethylthiocarbonyl)-phenylimino]-8-thia1,6-diazabicyclo[4.3.0]nonan-7-on (Verb. No. 1.52).

Besonders herausragend ist 9-[4-Chlor-2-fluor-5-(2-methoxy-ethoxycarbonyl-1-ethylthiocarbonyl)-phenylimino]-8thia1,6-diazabicyclo[4.3.0]nonan-7-on (Verb. No. 1.54).

Weiterhin betrifft die Erfindung Verbindungen der Formel Ic,

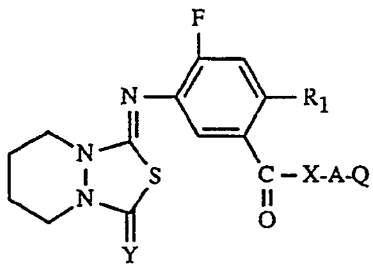

(Ic),

worin

Y Sauerstoff;

X Sauerstoff; oder Schwefel;

$R_1$ Chlor,

A eine $C_1$-$C_2$-Alkylenbrücke; und

Q einen über Kohlenstoff oder Stickstoff gebundenen 5- oder 6-gliedrigen Heterocyclus, welcher 1 bis 3 Heteroatome ausgewählt aus der Gruppe N, O und S enthält, wobei diese Heterocyclen ihrerseits noch benzanelliert sein und bis zu zweifach durch Halogen, $C_1$-$C_4$-Alkyl, $C_1$-$C_3$-Halogenalkyl, $C_1$-$C_3$-Alkoxy, $C_1$-$C_3$-Halogenalkoxy, $C_1$-$C_3$-Dialkylamino, Hydroxy oder Carbonylgruppen substituiert sein können

bedeutet.

Insbesondere betrifft die Erfindung Verbindungen der Formel Ic,

worin

Y Sauerstoff;

X Sauerstoff;

A $-CH_2-$; $-CH_2-CH_2-$; oder $-CH(CH_3)-$; und

Q einen gegebenenfalls bis zu zweifach durch $C_1$-$C_4$-Alkyl oder einfach durch eine Carbonylgruppe substituierten heterocyclischen Rest ausgewählt aus der Gruppe Pyridinyl, 1,3-Thiazolyl, Thiophenyl, Pyrrolidinyl, Morpholinyl, Furanyl, Indolyl, Pyrazolyl, 1,2-Oxazolyl, Pyrrolyl, Imidazolyl, Pyrazolyl und 1,2,4-Triazolyl; bedeutet.

Als besonders bevorzugte Einzelverbindungen der Formel Ic sind zu nennen:

9-{4-Chlor-2-fluor-5-[2-(4-methyl-thiazol-5-yl)-ethoxyarbonyl]-phenylimino}-8-thia1,6-diazabicyclo[4.3.0]nonan-7-on (Verb. No. 1.21) und

9-{4-Chlor-2-fluor-5-[2-(pyridin-2-yl)-ethoxyarbonyl]-phenylimino}-8-thia1,6-diazabicyclo[4.3.0]nonan-7-on (Verb. No. 1.20).

Weiterhin betrifft die Erfindung Verbindungen der Formel Id,

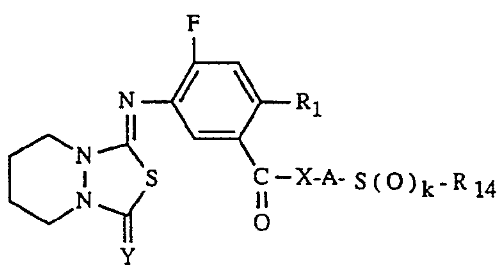

(Id),

worin

Y Sauerstoff;

X Sauerstoff; oder Schwefel;

$R_1$ Chlor;

A eine $C_2$-$C_4$-Alkylenbrücke;

k 0; und

$R_{14}$ $C_1$-$C_{10}$-Alkyl;

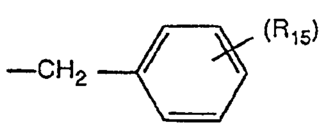 ; oder 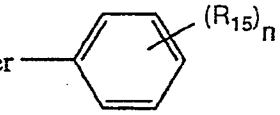 ; und

$R_{15}$ $C_1$-$C_4$-Alkyl; Halogen; oder $C_1$-$C_4$-Alkoxy;
bedeutet.

Insbesondere betrifft die Erfindung Verbindungen der Formel Id, worin

X Sauerstoff;
A -$CH(CH_3)$-$CH_2$-; oder -$CH_2$-$CH_2$-;
k 0;
$R_{14}$ $C_1$-$C_8$-Alkyl; oder

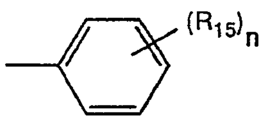 ;

und

$R_{15}$ $C_1$-$C_4$-Alkyl; Halogen; oder $C_1$-$C_4$-Alkoxy;
bedeutet.

Besonders gut wirksam sind auch Verbindungen der Formel Id, worin

A 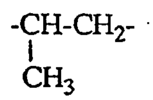

und Q $C_1$-$C_5$-Alkylthio bedeuten. Von diesen Verbindungen sind besonders diejenigen bevorzugt, in denen X und Y für Sauerstoff stehen.

Namentlich zu nennen sind die folgenden Verbindungen der Formel Id:

9-[4-Chlor-2-fluor-5-(2-methylthio-1-methylethoxyarbonyl)-phenylimino]-8-thia1,6-diazabicyclo[4.3.0] nonan-7-on (Verb. No. 1.02),

9-[4-Chlor-2-fluor-5-(2-ethylthio-1-methylethoxyarbonyl)-phenylimino]-8-thia1,6-diazabicyclo[4.3.0]nonan-7-on (Verb. No. 1.03),

9-[4-Chlor-2-fluor-5-(2-propylthio-1-methylethoxyarbonyl)-phenylimino]-8-thia1,6-diazabicyclo[4.3.0]nonan-7-on (Verb. No. 1.04),

9-[4-Chlor-2-fluor-5-(2-i-propylthio-1-methylethoxyarbonyl)-phenylimino]-8-thia1,6-diazabicyclo[4.3.0] nonan-7-on (Verb. No. 1.05),

9-[4-Chlor-2-fluor-5-(2-n-butylthio-1-methylethoxyarbonyl)-phenylimino]-8-thia1,6-diazabicyclo[4.3.0] nonan-7-on (Verb. No. 1.06),

9-[4-Chlor-2-fluor-5-(2-phenylthio-ethoxyarbonyl)-phenylimino]-8-thia1,6-diazabicyclo-[4.3.0]nonan-7-on (Verb. No. 1. 16) und

9-[4-Chlor-2-fluor-5-(2-p-chlorphenylthio-1-methyl-ethoxyarbonyl)-phenylimino]-8-thia1,6-diazabicyclo [4.3.0]nonan-7-on (Verb. No. 1.56).

Die Verbindungen der Formel I werden hergestellt, indem man ein Isothiocyanato-benzoesäureester der Formel II

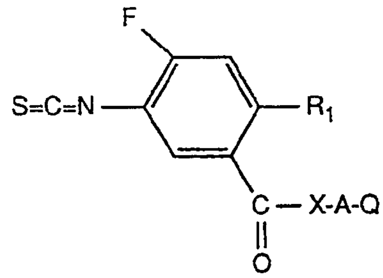

(II),

worin $R_1$, X, A und Q die unter der Formel I angegebene Bedeutung haben, mit Hexahydropyridazin in die Verbindung der Formel III

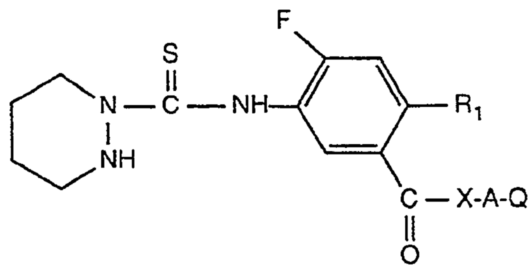

(III)

überführt und diese anschliessend mit einer Verbindung der Formel IV

$CYCl_2$ (IV),

worin Y für Sauerstoff oder Schwefel steht, in Gegenwart einer Base umsetzt.

Die Isothiocyanato-benzoesäureester der Formel II und die Thioharnstoffe der Formel III sind neu. Sie sind wertvolle Zwischenverbindungen für die Herstellung der erfindungsgemässen Endprodukte. Sie sind ebenfalls Gegenstand dieser Anmeldung.

Die Isothiocyanato-benzoesäureester der Formel II

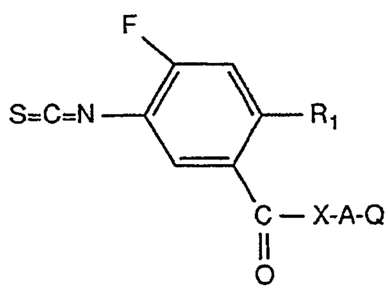

(II),

können hergestellt werden, durch Umsetzung eines Anilins der Formel X

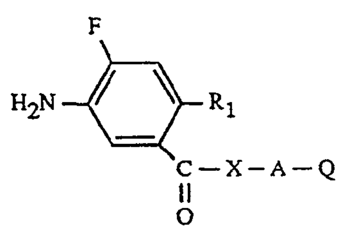

(X),

worin $R_1$, X, A und Q wie zuvor definiert sind, mit Thiophosgen.

Die Umsetzung der Benzoesäureester der Formel II mit Hexahydropyridazin wird mit Vorteil in einem reaktionsinerten Lösungsmittel bei Temperaturen von -5°C bis zur Siedetemperatur des Lösungsmittels, insbesondere von 0 bis +50°C, besonders bevorzugt bei Raumtemperatur, durchgeführt. Geeignete Lösungsmittel für diese Reaktion sind beispielsweise Toluol, Xylol, Essigsäureethylester oder Acetonitril.

Die Umsetzung der Verbindung der Formel III mit der Verbindung der Formel IV erfolgt vorteilhaft in einem reaktionsinerten Lösungsmittel bei niedrigen Temperaturen, vorzugsweise bei 0 bis +50°C, besonders bevorzugt bei 0 bis +15°C. Geeignete Basen für diese Reaktion sind beispielsweise Pyridin, Triethylamin oder N,N-Dimethylanilin.

Als Lösungsmittel eignen sich beispielsweise 1,2-Dichlorethan, Dichlormethan oder Toluol.

Nach einem weiteren Verfahren können die Verbindungen der Formel I durch Umsetzung einer Verbindung der Formel VI

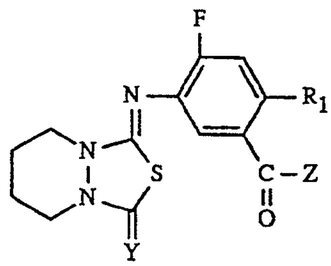

(VI),

worin $R_1$ und Y wie unter Formel I definiert sind und Z für eine nucleofuge Gruppe, wie Chlor, Brom, Azido oder -O-CO-$C_1$-$C_4$-Alkyl oder für OH steht, mit einem Alkohol oder Thiol der Formel VII

H - X - A - Q (VII)

worin X, A und Q wie unter Formel I definiert sind.

Durch Veresterungsreaktion darstellbar sind auch die Verbindungen der Formel I'

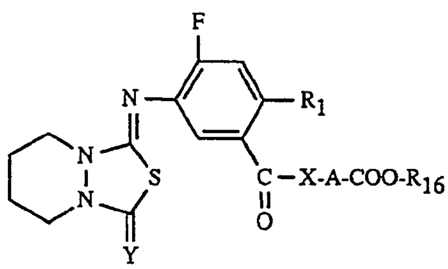

(I')

worin die Reste X, Y, $R_1$, $R_{16}$ und A wie unter Formel I definiert sind durch Umsetzung eines Carbonsäurederivats der Formel VIII

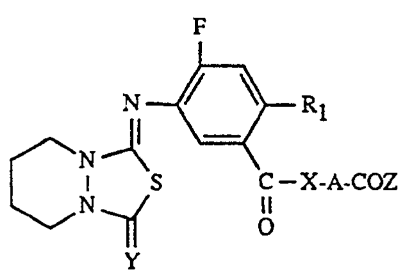

(VIII),

worin die Reste X, Y, $R_1$ und A wie unter Formel I definiert sind und Z für eine nucleofuge Gruppe, wie Chlor, Brom, Azido oder -O-CO-$C_1$-$C_4$-Alkyl oder für OH steht, mit einem Alkohol oder Thiol der Formel IX

HO - $R_{16}$ (IX)

worin $R_{16}$ wie unter Formel I definiert sind.

Die Veresterung der Säuren VI oder VIII (Z = OH) wird unter Säurekatalyse in einem inerten Lösungsmittel oder direkt in dem Alkohol oder Thialkohol VII durchgeführt. Bei der Veresterung der Säurederivate der Formeln VI oder VIII, in denen Z für eine nucleofuge Gruppe steht, ist der Zusatz einer katalytischen oder äquimolaren Menge einer Base vorteilhaft. Derartige Veresterungsreaktionen sind dem Fachmann geläufig.

Die Alkohole der Formeln VII und IX sind bekannt oder können analog zu literaturbekannten Verfahren hergestellt werden. Insbesondere die Alkohole VII, in denen Q für ein Heterocyclus steht können durch Reduktion der entsprechenden Carbonsäuren, Aldehyde oder Ketone oder aber durch die Addition des Heterocyclus an

entsprechend substituierte Oxirane hergestellt werden. Insbesondere ist für die Darstellung dieser Alkohole auf die folgende Literatur hinzuweisen:

Pyridine:E. Brown (ed.), *The chemistry of Herterocyclic Compounds*, Vol. 14, part 4: Pyridine and its Derivatives, J. Wiley and Sons, 1964, Seiten 2 bis 100.

1,3-Thiazole: J.P. Aune in *The chemistry of Herterocyclic Compounds*, Vol. 34, part 1: Thiazole and its Derivatives, J. Wiley and Sons, 1979, Seiten 452 bis 459.

Thiophene: G. Masumarra in *The chemistry of Herterocyclic Compounds*, Vol. 44, part 3: Thiophene and its Derivatives, J. Wiley and Sons, 1986, Seiten 975 bis 1131.

Pyrroline: H.J. Anderson und C.E. Loader in *The chemistry of Herterocyclic Compounds*, Vol. 48: Pyrroline and its Derivatives, J. Wiley and Sons, 1990, Seiten 398 bis 483.

1,2,4-Triazole: C. Temple in *The chemistry of Herterocyclic Compounds*, Vol. 37: 1,2,4-Triazoles and its Derivatives, J. Wiley and Sons, 1981, Seiten 3 bis 29 und *Beilsteins Handbuch der Organischen Chemie*, Bd. $26^{III/IV}$, Seiten 36 bis 326.

Imidazole: K. Hofmann (Ed.) *The chemistry of Herterocyclic Compounds*, Vol. 6: Imidazoles and its Derivatives, J. Wiley and Sons, 1953, Seiten 340 bis 360; *Beilsteins Handbuch der Organischen Chemie*, Bd. $23^{III/IV}$ Seite 571.

Furane: *Rodd's chemistry of Carbon Compounds*, Vol IV, Part A, Seiten 109 ff, Elsevier Sci. Publ. Comp. Amsterdam 1973.

Morpholine: *Beilstein's Handbuch der Organischen Chemie*, Bd. $27^{III/IV}$, Seiten 56, 121 ff und 201.

Pyrrolidine: *Beilstein's Handbuch der Organischen Chemie*, Bd. $20^{III/IV}$, Seiten 92, 114, 124 und 125.

Pyrrolidinone: *Beilstein's Handbuch der Organischen Chemie*, Bd. $21^{III/IV}$, Seiten 3152 ff und 3259 ff.

Indole: Chem. Abstr. <u>112</u>, 098378 und Chem. Abstr. <u>111</u>, 042668

Die Verbindungen der Formel Ie

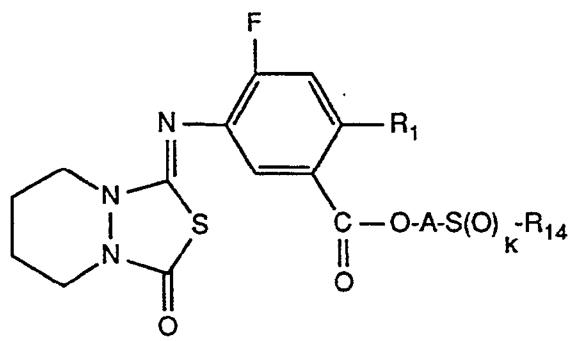

(Ie),

worin $R_1$, $R_{14}$ und A wie unter Formel I in Anspruch I definiert sind und k für 1 oder 2 steht, können auch erhalten werden, indem man einen Thioether der Formel If

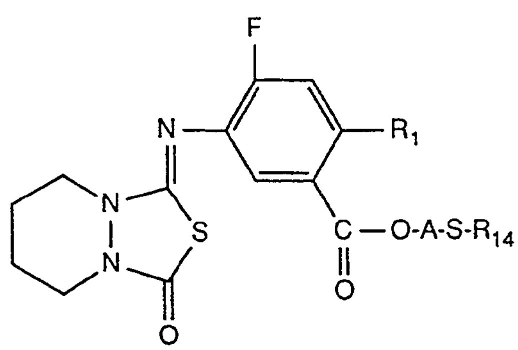

(If),

worin $R_1$, $R_{14}$ und A die unter Formel I in Anspruch I angegebene Bedeutung haben, oxidiert.

Die Oxidationen werden vorzugsweise in Lösung bei Temperaturen von 0°C bis 100°C durchgeführt.

Derartige Oxidationen sind dem Fachmann geläufig. Sie sind unter anderem beschrieben in Houben Weyl, Methoden der Organischen Chemie, Bd. IX, S. 211 ff. Geeignete Oxidationsmittel sind neben Wasserstoffperoxid, oder Persäuren, wie etwa m-Chlorperbenzoesäure, Kaliumpermanganat in Eisessig.

Die Ausgangsverbindungen der Formel II und IV sind bekannt oder können analog zu literaturbekannten Verfahren hergestellt werden. Beispielsweise kann man die Verbindungen der Formel II erhalten, indem man

die entsprechenden Amino-benzoesäureester der Formel V

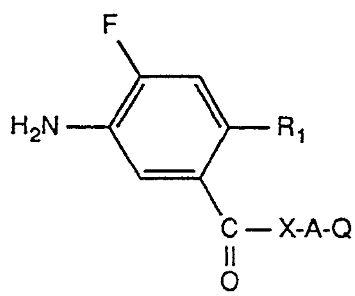

(V)

worin $R_1$, X, A und Q die unter Formel I angegebene Bedeutung haben, mit Thiocarbonylchlorid umsetzt.

Die Herstellung der Ausgangsverbindungen der Formel V sind in der EP-A-0233151 und EP-A-0338987 beschrieben.

Die Ausgangsverbindungen der Formeln VI und VIII können analog zu literaturbekannten Verfahren gemäss nachstehendem Schema hergestellt werden:

SCHEMA 1

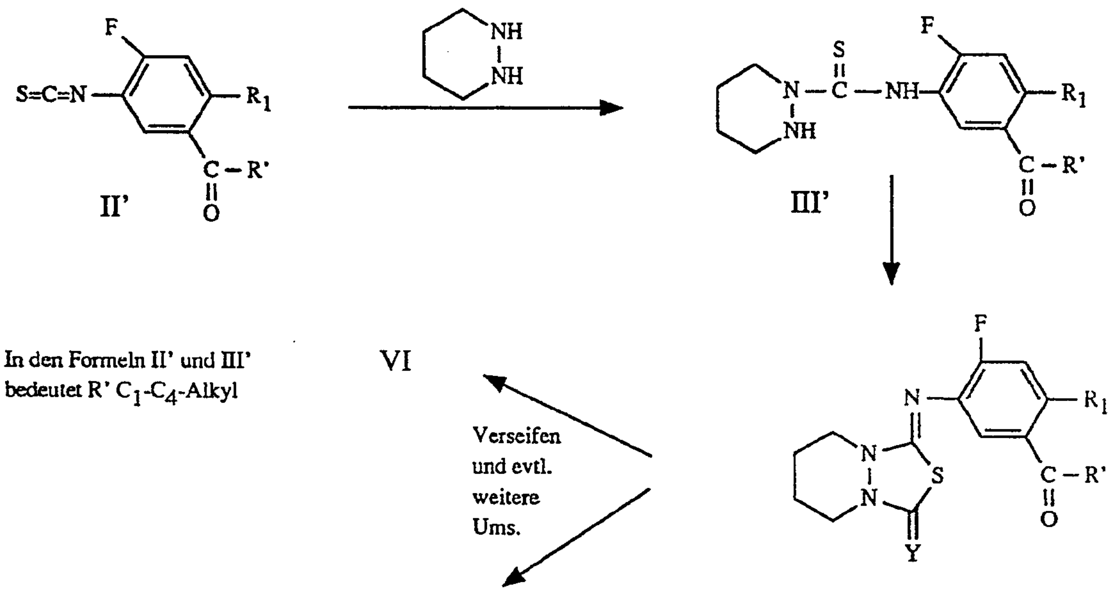


II'

III'

VI

VIII

In den Formeln II' und III' bedeutet R' $C_1$-$C_4$-Alkyl

Ausgehend von dem Ester II' wird zunächst, analog zur Synthese der Verbindungen der Formel I der Thioharnstoff III' hergestellt. Diser wird dann zu dem 9-Phenylimino-8-thia-1,6-diazabicyclo[4.3.0]nonan-7-on-Ester cyclisiert. Aus diesem Ester kann durch Verseifen die freie Säure hergestellt werden. Ueber die Säure (R'= OH) sind die Säurechloride, -bromide, -azide oder gemischten Säureanhydride der Formeln VI und VIII nach an sich bekannten Verfahren leicht zugänglich.

Die Wirkstoffe der Formel I werden in der Regel mit Aufwandmengen von 0,001 bis 5 kg/ha insbesondere 0,005 bis 3 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und selektiv herbizide Eigenschaften aus, die sie ausgezeichnet zum Einsatz in Kulturen von Nutzpflanzen, insbesondere in Getreide, Baumwolle, Soja, Raps, Mais und Reis befähigen. Bevorzugt ist die Verwedung als Selektivherbizid in Getreide (Weizen, Gerste, Roggen), Soja und Mais, insbesondere in Soja.

Die Erfindung betrifft auch herbizide und pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemässen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Strassenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkung erreicht man in Raps, Sonnenblumen, Mais und anderen Kulturpflanzen. Des weiteren kann durch Hemmung des vegetativen Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodass die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Für die Verwendung der Verbindung der Formel I oder sie enthaltender Mittel als Herbizid oder zur Regulierung des Pflanzenwachstums kommen verschiedene Methoden und Techniken in Betracht, wie beispielsweise die folgenden:

i) <u>Samenbeizung</u>

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei bis zu 4 g Wirkstoff der Formel I (bei einer 50%-igen Formulierung: bis zu 8,0 g Spritzpulver) pro 1 kg Saatgut.

b) Beizung der Samen mit einem Emulsionskonzentrat des Wirkstoffs oder mit einer wässrigen Lösung des als Spritzpulver formulierten Wirkstoffs der Formel I nach Methode a) (Nassbeizung).

c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 4,0 g bis 0,001 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) <u>Kontrollierte Wirkstoffabgabe</u>

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykolethergruppen und 10 bis 100 Propylenglykolethergruppen enthaltenden Polyethylenoxidaddukte an Polypropylenglykol, Aethylendiaminopolypropylenglykol und Alkylpolypropylenglykol mit I bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyethylenglykolether, Polypropylen-polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chlorethyl)-ethylammoniumbromid.

Die in der Formulierungstechnik gebäuchlichen Tenside sind u.a. in folgenden Publikationen beschrieben:

- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die agrochemischen Zubereitungen enthalten in der Regel 0,1 bis 95 %, insbesondere 0,1 bis 80 % Wirk-

stoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

Emulgierbare Konzentrate:

| | |
|---|---|
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssiges Trägermittel: | 50 bis 94 %, vorzugsweise 70 bis 85 % |

Stäubemittel:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspensions-Konzentrate:

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbares Pulver:

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 99 %, vorzugsweise 15 bis 90 % |

Granulate:

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 5 kg AS/ha, vorzugsweise 0,005 bis 3 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele erläutern die Erfindung.

Herstellungsbeispiele:

Beispiel H1: Herstellung von 2-Chlor-4-fluor-5-nitro-benzoesäure-(1-methyl-2-methylthio)-ethylester

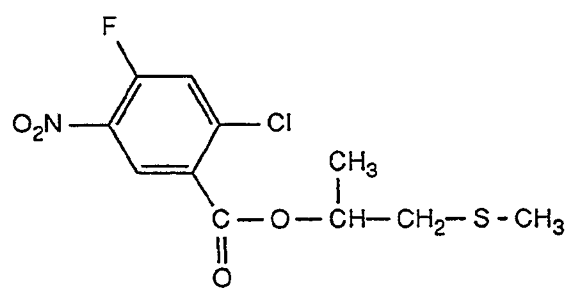

Zu einer Lösung von 9,3 g Methylthiopropan-2-ol und 8,8 g Triethylamin in 100 ml Essigsäureethylester tropft man unter Rühren bei Raumtemperatur eine Lösung von 20,7 g 2-Chlor-4-fluor-5-nitrobenzoesäurechlorid in 25 ml Essigsäureethylester hinzu. Nach der Zugabe wird noch weitere 7 Stunden bei Raumtemperatur gerührt, vom entstandenen Triethylamin-Hydrochlorid abfiltriert und das Filtrat im Vakuum eingeengt.

Man erhält 21,4 g 2-Chlor-4-fluor-5-nitro-benzoesäure-(1-methyl-2-methylthio)-ethylester in fester Form mit einem Schmelzpunkt +47 bis +48°C.

Beispiel H2: Herstellung von 5-Amino-2-chlor-4-fluor-benzoesäure-(1-methyl-2-methylthio)-ethylester

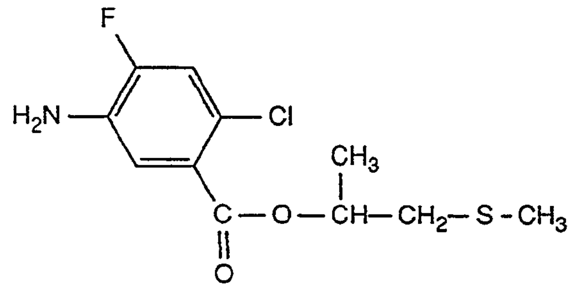

21,4 g gemäss Beispiel H1 erhaltenen 2-Chlor-4-fluor-5-nitro-benzoesäure-(1-methyl-2-methylthio)-ethylesters werden in 240 ml Tetrahydrofuran bei einer Temperatur von 20-25°C in Gegenwart von 14 g Raney-Nickel-Katalysator unter Normaldruck mit Wasserstoff hydriert. Nachdem die stöchiometrische Menge Wasserstoff verbraucht worden ist, wird der Katalysator abgetrennt und die Lösung eingedampft. Man erhält 18,6 g 5-Amino-2-chlor-4-fluor-benzoesäure-(1-methyl-2-methylthio)-ethylester, $n_D^{22}$ 1,5628.

Beispiel H3: Herstellung von 2-Chlor-4-fluor-5-isothiocyanato-benzoesäure-(1-methyl-2-methylthio)-ethylester

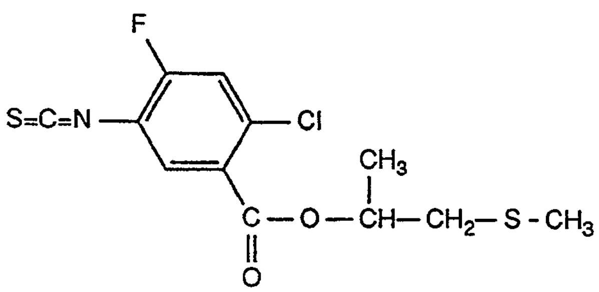

Zu einer Suspension von 2,3 g Calciumcarbonat, 1,5 ml Thiophosgen, 10 ml Dichlormethan und 10 ml Wasser tropft man eine Lösung von 4,2 g gemäss Beispiel H2 erhaltenen 5-Amino-2-chlor-4-fluor-benzoesäure-( 1-methyl-2-methylthio)-ethylesters in 20 ml Dichlormethan bei 25-30°C hinzu. Nach Beendigung der Kohlendioxydentwicklung lässt man 18 Stunden lang bei Raumtemperatur weiterrühren. Nach Filtration und Waschen mit Wasser trennt man die organische Phase ab, trocknet sie über Natriumsulfat und dampft anschliessend ein. Man erhält 5,4 g 2-Chlor-4-fluor-5-isothiocyanato-benzoesäure(1-methyl-2-methylthio)-ethylester in öliger Form, das ohne weitere Reinigung im nachfolgenden Reaktionsschritt eingesetzt wird.

Beispiel H4: Herstellung von 2-Chlor-4-fluor-5-(1-hexahydropyridazinyl-thiocarbonylamino)-benzoesäure-(1-methyl-2-methylthio)-ethylester

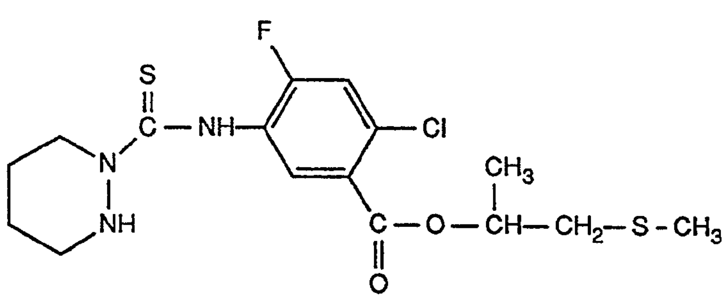

Zu einer Lösung von 1,2 g Hexahydropyridazin in 5 ml Toluol tropft man unter Rühren bei 20-25°C eine Lösung von 4,4 g gemäss Beispiel H3 erhaltenen 2-Chlor-4-fluor-5-isothiocyanato-benzoesäure-(1-methyl-2-methylthio)-ethylester in 10 ml Toluol hinzu. Anschliessend wird weitere 2 Stunden bei Raumtemperatur gerührt. Nach dem Eindampfen im Vakuum erhält man 5,4 g 2-Chlor-4-fluor-5-(1-hexahydropyridazinyl-thiocarbonylamino)-benzoesäure-(1-methyl-2-methylthio)-ethylester, $n_D^{22}$ 1,6018.

Beispiel H5: Herstellung von 9-[4-Chlor-2-fluor-5-(1-methyl-2-methylthio-ethoxycarbonyl)-phenylimino]-8-thia-1,6-diazabicyclo[4.3.0]nonan-7-on (Verbindung Nr. 1.02) :

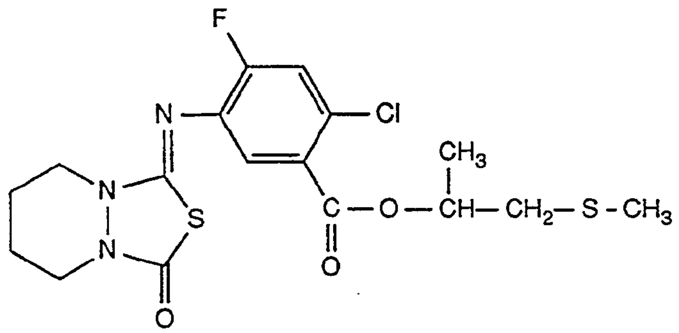

(Verbindung Nr. 1.02)

Zu einer Lösung von 5,2 g gemäss Beispiel H4 erhaltenen 2-Chlor-4-fluor-5-(1-hexahydropyridazinyl-thiocarbonylamino)-benzoesäure-(1-methyl-2-methylthio)-ethylesters und 4 ml Pyridin in 100 ml Dichlormethan tropft man unter Rühren bei einer Temperatur von 0°C 1 ml 20%ige toluolische Lösung von Phosgen hinzu. Anschliessend wird weitere 2 Stunden gerührt und dann das Reaktionsgemisch in Eis/Wasser gegossen. Die organische Phase wird abgetrennt und über Natriumsulfat getrocknet. Nach dem Eindampfen und anschliessender chromatographischer Reinigung des erhaltenen öligen Produktes erhält man 3,2 g 9-[4-Chlor-2-fluor-5-(1-methyl-2-methylthio-ethoxycarbonyl)-phenylimino]-8-thia-1,6-diazabicyclo[4.3.0]nonan-7-on (Verbindung Nr. 1.02), $n_D^{22}$ 1,5973.

Beispiel H6: Herstellung von 2-Chlor-4-fluor-5-nitro-thiolbenzoesäure-(methoxy-carbonylmethyl)-ester

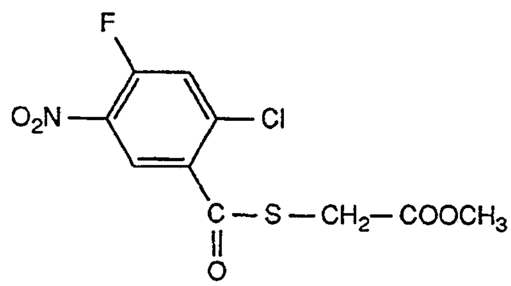

Zu einer Lösung von 20 ml Thioglycolsäuremethylester in 100 ml Essigsäureethylester gibt man unter Rühren bei Raumtemperatur 48 g 2-Chlor-4-fluor-5-nitro-benzoesäurechlorid tropfenweise hinzu. Nach 18-stündigem Rühren und anschliessendem Abdampfen der Lösung unter Vakuum erhält man 19 g 2-Chlor-4-fluor-5-nitro-thiolbenzoesäure(methoxycarbonyl)-ester in Form eines Öles mit $n_D^{23}$: 1,5709.

Beispiel H7: Herstellung von5-Amino-2-chlor-4-fluor-thiolbenzoesäure-(methoxycarbonylmethyl)-ester

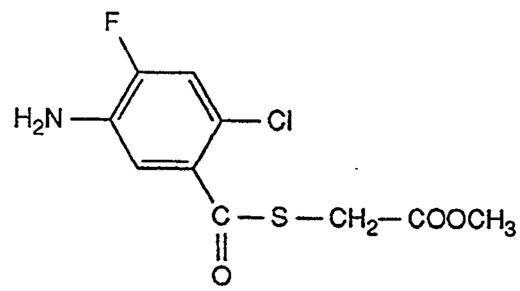

10,4 g gemäss Beispiel H1 erhaltenen 2-Chlor-4-fluor-5-nitro-thiolbenzoesäure-(methoxycarbonylmethyl)-esters werden in 150 ml Tetrahydrofuran bei einer Temperatur von 20-25°C in Gegenwart von 2 g Raney-Nickel-Katalysator unter Normaldruck mit Wasserstoff hydriert. Nachdem die stöchiometrische Menge Wasserstoff verbraucht worden ist, wird der Katalysator abgetrennt und die Lösung eingedampft. Man erhält 8,6 g 5-Amino-2-chlor-4-fluor-thiolbenzoesäure-(methoxycarbonylmethyl)-ester mit einem Schmelzpunkt von +88 bis +89°C.

Beispiel H8: Herstellung von 2-Chlor-4-fluor-5-isothiocyanato-thiolbenzoesäure-(methoxycarbonylmethyl)-ester

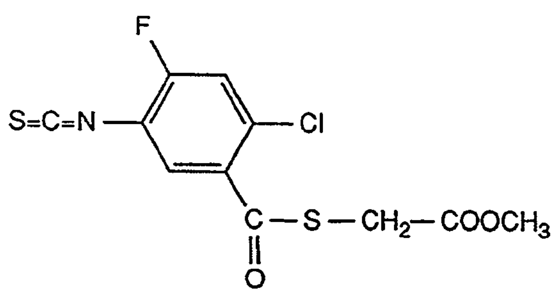

Zu einer Suspension von 6 g Calciumcarbonat, 4 ml Thiophosgen, 20 ml Dichlormethan und 20 ml Wasser tropft man eine Lösung von 3,8 g gemäss Beispiel H2 erhaltenen 5-Amino-2-chlor-4-fluor-thiolbenzoesäure-(methoxycarbonylmethyl)-esters in 100 ml Dichlormethan bei 25-30°C hinzu. Nach Beendigung der Kohlendioxydentwicklung lässt man 18 Stunden lang bei Raumtemperatur weiterrühren. Nach Filtration und Waschen mit Wasser trennt man die organische Phase ab, trocknet sie über Natriumsulfat und dampft anschliessend ein. Man erhält 4,8 g 2-chlor-4-fluor-5-isothiocyanato-thiolbenzoesäure-(methoxycarbonylmethyl)-esters in Form eines Öles, das ohne weitere Reinigung im nachfolgenden Reaktionsschritt eingesetzt wird.

Beispiel H9: Herstellung von 2-Chlor-4-fluor-5-(1-hexahydropyridazinyl-thiocarbonylamino)-thiolbenzoesäure-(methoxycarbonylmethyl)-ester

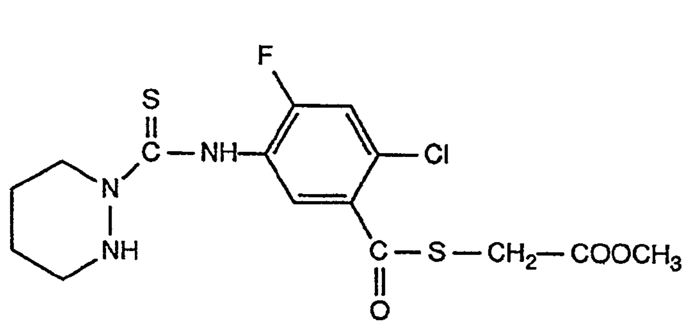

Zu einer Lösung von 1,4 g Hexahydropyridazin in 20 ml Toluol tropft man unter Rühren bei 20-25°C eine Lösung von 4,8 g gemäss Beispiel H3 erhaltenen 2-Chlor-4-fluor-5-isothiocyanato-thiolbenzoesäure-(methoxycarbonylmethyl)-ester in 50 ml Toluol hinzu. Anschliessend wird weitere 8 Stunden bei Raumtemperatur gerührt. Nach dem Eindampfen im Vakuum erhält man 4,4 g 2-Chlor-4-fluor-5-(1-hexahydropyridazinyl-thiocarbonylamino)-thiolbenzoesäure-(methoxycarbonylmethyl)-ester in harziger Form.

Beispiel H10: Herstellung von 9-[4-Chlor-2-fluor-5-(methoxycarbonylmethylthiocarbonyl)-phenylimino]-8-thia-1,6-diazabicyclo[4.3.0]nonan-7-on (Verbindung Nr. 2.13):

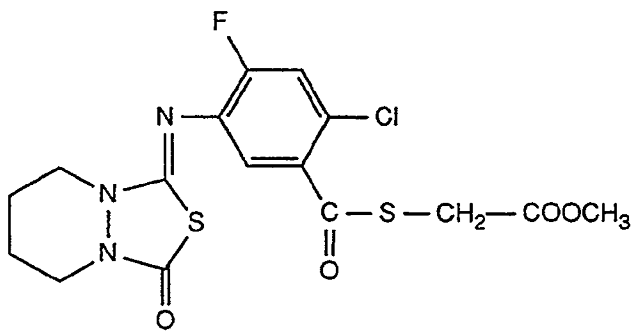

(Verbindung Nr. 2.13)

Zu einer Lösung von 4,4 g gemäss Beispiel H4 erhaltenen 2-Chlor-4-fluor-5-(1-hexahydropyridazinyl-thiocarbonylamino)-thiolbenzoesäure-(methoxycarbonylmethyl)-esters und 5 ml Pyridin in 10 ml Dichlormethan tropft man unter Rühren bei einer Temperatur von 0°C 8 ml 20%ige toluolische Lösung von Phosgen hinzu. Anschliessend wird weitere 2 Stunden gerürht und dann das Reaktionsgemisch in Eis/Wasser gegossen. Die organische Phase wird abgetrennt und über Natriumsulfat getrocknet. Nach dem Eindampfen und anschliessender chromatographischer Reinigung des erhaltenen öligen Produktes erhält man 3 g 9-[4-Chlor-2-fluor-5-(methoxycarbonylmethylthiocarbonyl)phenylimino]-8-thia-1,6-diazabicyclo[4.3.0]nonan-7-on (Verbindung Nr. 2.13), mit $n_D^{19}$ 1,5727.

Beispiel H10: Herstellung von 9-{4-Chlor-2-fluor-5-[2-(4-methyl-1,3-thiazol-5-yl)-ethoxycarbonyl]-phenylimino}-8-thia- 1,6-diazabicyclo[4.3.0]nonan-7-on (Verbindung Nr. 1.21 ):

Zu einer Lösung aus 1,2 ml 5-(2-Hydroxyethyl)-4-methylthiazol und 1,5 ml Triethylamin in 50 ml Essigsaäuremethylester gibt man unter Rühren bei Raumtemperatur eine Lösung von 3,6 g 9-[4-Chlor-2-fluor-5-(chlorcarbonyl)-phenylimino]-8-thia-1,6-diazabicyclo[4.3.0]nonan7-on in 10 ml Essigsäuremethylester tropfenweise hinzu. Nach 12-stündigem Rühren bei Raumtemperatur filiert man das ausgefallene Triethylamin Hydrochlorid ab und dampft das Filtrat ein. Man erhält 3,8 g der Titelverbindung als Oel mit $n_D^{22}$ 1,6030.

Analog zu vorstehenden Beispielen und den in der Beschreibung genannten Herstellungsverfahren können die Verbindungen der Tabellen 1 und 2 hergestellt werden.

Tabelle 1:

Verbindungen der Formel I

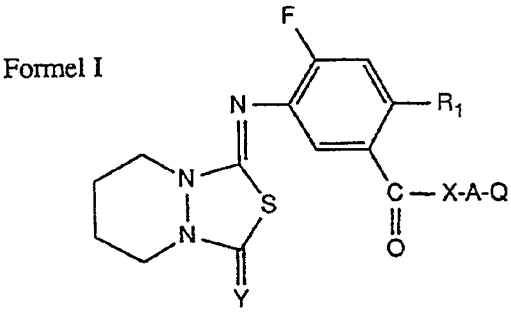

(I)

| Verb. Nr. | $R_1$ | X | Y | A*) | Q | physik. Daten |
|---|---|---|---|---|---|---|
| 1.01 | Cl | O | O | $-CH-CH_2-$ | $SCH_3$ | |
| 1.02 | Cl | O | O | $-CH(CH_3)-CH_2-$ | $SCH_3$ | $n_D^{22}$ 1,5973 |
| 1.03 | Cl | O | O | $-CH(CH_3)-CH_2-$ | $SC_2H_5$ | $n_D^{22}$ 1,5765 |
| 1.04 | Cl | O | O | $-CH(CH_3)-CH_2-$ | $SC_3H_7$ | $n_D^{23}$ 1,5825 |
| 1.05 | Cl | O | O | $-CH(CH_3)-CH_2-$ | $SC_3H_7(i)$ | $n_D^{23}$ 1,5768 |
| 1.06 | Cl | O | O | $-CH(CH_3)-CH_2-$ | $SC_4H_9$ | $n_D^{23}$ 1,5691 |
| 1.07 | Cl | O | O | $-CH(CH_3)-CH_2-$ | $SC_4H_9(s)$ | |
| 1.08 | Cl | O | O | $-CH(CH_3)-CH_2-$ | $SC_4H_9(t)$ | |
| 1.09 | Cl | O | O | $-CH(CH_3)-CH_2-$ | $SC_5H_{11}$ | |
| 1.10 | Cl | O | O | $-CH(CH_3)-CH_2-$ | $SC_6H_{13}$ | |
| 1.11 | Cl | O | O | $-CH(CH_3)-CH_2-$ | $SC_7H_{15}$ | |
| 1.12 | Cl | O | O | $-CH(CH_3)-CH_2-$ | $SC_8H_{17}$ | |

*) Die Alkylenbrücke A ist in Leserichtung (von links nach rechts) an die Substituenten X und Q gebunden

Tabelle 1 (Fortsetzung)

| Verb. Nr. | $R_1$ | X | Y | A | Q | physik. Daten |
|---|---|---|---|---|---|---|
| 1.13 | Cl | O | O | $-CH-CH_2-$<br>$CH_3$ | $SC_9H_{19}$ | |
| 1.14 | Cl | O | O | $-CH-CH_2-$<br>$CH_3$ | $SC_{10}H_{21}$ | |
| 1.15 | Cl | O | O | $-CH-CH_2-$<br>$CH_3$ | 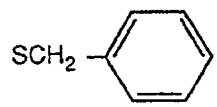 | |
| 1.16 | Cl | O | O | $-CH_2-CH_2-$ | 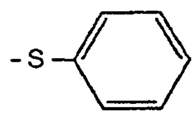 | $n_D^{22}$ 1,6174 |
| 1.17 | Cl | O | O | $-CH-CH_2-$<br>$CH_3$ | 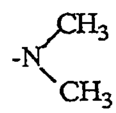 | $n_D^{22}$ 1,5802 |
| 1.18 | Cl | O | O | $-CH-CH_2-$<br>$CH_3$ | 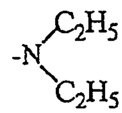 | $n_D^{22}$ 1,5635 |
| 1.19 | Cl | O | O | $-CH_2-CH_2-$ | 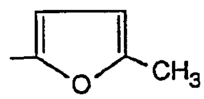 | |
| 1.20 | Cl | O | O | $-CH_2-CH_2-$ | 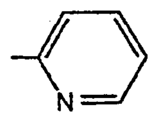 | $n_D^{22}$ 1,6076 |
| 1.21 | Cl | O | O | $-CH_2-CH_2-$ | 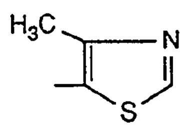 | $n_D^{22}$ 1,6030 |
| 1.22 | Cl | O | O | $-CH_2-CH_2-$ | 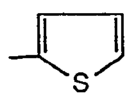 | |
| 1.23 | Cl | O | O | $-CH_2-CH_2-$ | 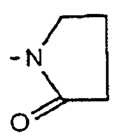 | $n_D^{22}$ 1,5859 |
| 1.24 | Cl | O | O | $-CH_2-CH_2-$ | 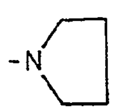 | |

Tabelle 1 (Fortsetzung)

| Verb. Nr. | $R_1$ | X | Y | A | Q | physik. Daten |
|---|---|---|---|---|---|---|
| 1.25 | Cl | O | O | $-CH_2-CH_2-$ | 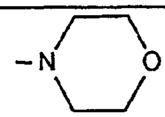 | Smp. 98 - 99°C |
| 1.26 | Cl | O | O | $-CH-CH_2-$<br>$CH_3$ | 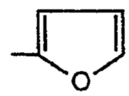 | |
| 1.27 | Cl | O | O | $-CH_2-CH_2-$ | 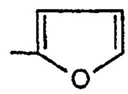 | |
| 1.28 | Cl | O | O | $-CH-CH_2-$<br>$CH_3$ | 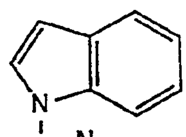 | |
| 1.29 | Cl | O | O | $-CH_2-CH_2-$ | 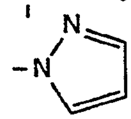 | |
| 1.30 | Cl | O | O | $-CH_2-CH_2-$ | 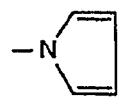 | |
| 1.31 | Cl | O | O | $-CH_2-CH_2-$ | 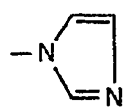 | |
| 1.32 | Cl | O | O | $CH_3$<br>$-CH-CH_2-$ | 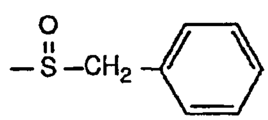 | |
| 1.33 | Cl | O | O | $CH_3$<br>$-CH-CH_2-$ | 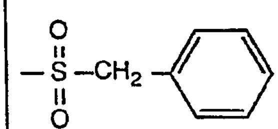 | |
| 1.34 | Cl | O | O | $CH_3$<br>$-CH-CH_2-$ | 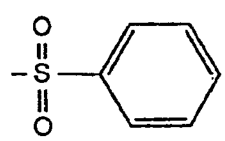 | |
| 1.35 | Cl | O | O | $CH_3$<br>$-CH-CH_2-$ | 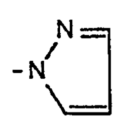 | |
| 1.36 | Cl | O | O | $CH_3$<br>$-CH-CH_2-$ | 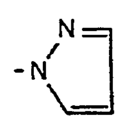 | |

Tabelle 1 (Fortsetzung)

| Verb. Nr. | $R_1$ | X | Y | A | Q | physik. Daten |
|---|---|---|---|---|---|---|
| 1.37 | Cl | O | O | $CH_3$<br>$-CH-CH_2-$ | 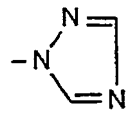 | |
| 1.38 | Cl | O | O | $CH_3$<br>$-CH-CH_2-$ | 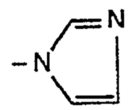 | |
| 1.39 | Cl | O | O | $-CH-CH_2-$<br>$CH_3$ | 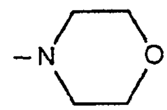 | |
| 1.40 | Cl | O | O | $-CH_2-$ | $-COOCH_2-CH_2-O-CH_3$ | |
| 1.41 | Cl | O | O | $-CH_2-$ | -COO 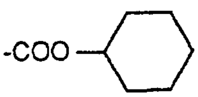 | |
| 1.42 | Cl | O | O | $-CH_2-$ | $-COOCH_2-CH_2-S-CH_3$ | |
| 1.43 | Cl | O | O | $-CH_2-$ | $CH_3$<br>$-COO-CH-CH_2-N$<br>$CH_3$ $CH_3$ | |
| 1.44 | Cl | O | O | $-CH-(CH_3)-$ | $-COO-CH_2-CH_2-O-CH_3$ | |
| 1.45 | Cl | O | O | $-CH-(CH_3)-$ | -COO 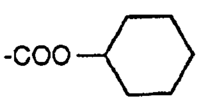 | |
| 1.46 | Cl | O | O | $-CH-(CH_3)-$ | $-COO-CH_2-CH_2-S-CH_3$ | |
| 1.47 | Cl | O | O | $-CH-(CH_3)-$ | $CH_3$<br>$COO-CH-CH_2-N$<br>$CH_3$ $CH_3$ | |
| 1.48 | Cl | S | O | $-CH_2-$ | -COO 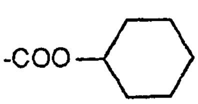 | $n_D^{28}$ 1,5272 |
| 1.49 | Cl | S | O | $-CH_2-$ | $COO-CH_2-CH_2-O-CH_3$ | $n_D^{28}$ 1.6038 |
| 1.50 | Cl | O | O | $-CH-CH_2-$<br>$CH_3$ | $-S-CH_2-COOC_2H_5$ | $n_D^{21}$ 1,5786 |

Tabelle 1 (Fortsetzung)

| Verb. Nr. | $R_1$ | X | Y | A | Q | physik. Daten |
|---|---|---|---|---|---|---|
| 1.51 | Cl | S | O | $-CH_2-$ | $CH_3$<br>$-COO-CH$<br>$CH_2-O-CH_3$ | $n_D^{20}$ 1,5911 |
| 1.52 | Cl | S | O | $-CH_2-$ | $-COO-CH_2-CH_2$<br>$O-C_2H_5$ | $n_D^{20}$ 1,5808 |
| 1.53 | Cl | S | O | $CH_3$<br>$-CH-$ | $-COOCH_2-CH_2-O-CH_3$ | $n_D^{20}$ 1,5578 |
| 1.54 | Cl | S | O | $CH_3$<br>$-CH-$ | $-COOCH_2-CH_2-O-C_2H_5$ | $n_D^{20}$ 1,5870 |
| 1.55 | Cl | S | O | $CH_3$<br>$-CH-$ | $CH_3$<br>$-COO-CH-CH_2-O-CH_3$ | $n_D^{20}$ 1,5796 |
| 1.56 | Cl | O | O | $CH_3$<br>$-CHCH_2-$ | 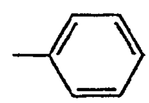 | |
| 1.57 | Cl | O | O | $-CHCH_2-$<br>$CH_3$ | 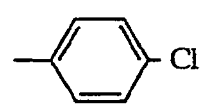 | $n_D^{22}$ 1,5997 |

Tabelle 2:

Verbindungen der Formel I

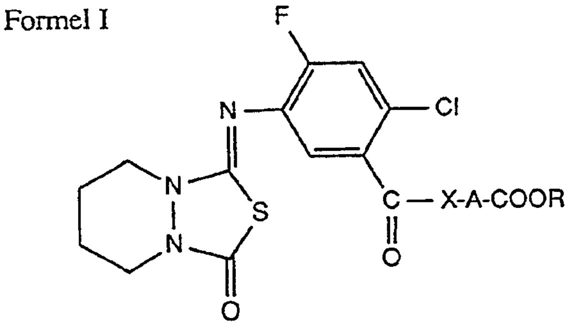

(I)

| Verb. Nr. | X | A | R | physik. Daten |
|---|---|---|---|---|
| 2.01 | O | $-CH_2-$ | $-COOCH_3$ | $n_D^{22}$ 1,5853 |
| 2.02 | O | $-CH_2-$ | $-COOC_2H_5$ | $n_D^{22}$ 1,5803 |
| 2.03 | O | $-CH_2-$ | $-COOC_5H_{11}(n)$ | |
| 2.04 | O | $-CH(CH_3)-$ | $-COOCH_3$ | mp. 87-88°C |
| 2.05 | O | $-CH(CH_3)-$ | $-COOC_2H_5$ | |
| 2.06 | O | $-CH(CH_3)-$ | $-COO-C_3H_7$ | |
| 2.07 | O | $-CH(CH_3)-$ | $-COOC_3H_7(i)$ | |
| 2.08 | O | $-CH(CH_3)-$ | $-COOC_4H_9$ | |
| 2.09 | O | $-CH(CH_3)-$ | $-COOC_4H_9(i)$ | |
| 2.10 | O | $-CH(CH_3)-$ | $-COOC_4H_9(s)$ | |
| 2.11 | O | $-CH(CH_3)-$ | $-COOC_4H_9(t)$ | |
| 2.12 | O | $-CH(CH_3)-$ | $-COOC_5H_{11}$ | |
| 2.13 | S | $-CH_2-$ | $-COOCH_3$ | $n_D^{19}$ 1,5727 |
| 2.14 | S | $-CH_2-$ | $-COOC_2H_5$ | $n_D^{22}$ 1,6118 |
| 2.15 | S | $-CH_2-$ | $-COOC_3H_7$ | |
| 2.16 | S | $-CH_2-$ | $-COOC_3H_7$ | |
| 2.17 | S | $-CH_2-$ | $-COOC_4H_9(i)$ | |
| 2.18 | S | $-CH_2-$ | $-COOC_4H_9(s)$ | |

Tabelle 2 (Fortsetzung)

| Verb. Nr. | X | A | R | physik. Daten |
|---|---|---|---|---|
| 2.19 | S | $-CH_2-$ | $-COOC_4H_9(t)$ | |
| 2.20 | S | $-CH_2-$ | $-COOC_4H_9$ | $n_D^{22}$ 1,5758 |
| 2.21 | S | $-CH(CH_3)-$ | $-COOCH_3$ | $n_D^{22}$ 1,6009 |
| 2.22 | S | $-CH(CH_3)-$ | $-COOC_2H_5$ | |
| 2.23 | S | $-CH(CH_3)-$ | $-COOC_3H_7$ | |
| 2.24 | S | $-CH(CH_3)-$ | $-COOC_3H_7(i)$ | |
| 2.25 | S | $-CH(CH_3)-$ | $-COOC_4H_9$ | |
| 2.26 | S | $-CH(CH_3)-$ | $-COOC_4H_9(i)$ | |
| 2.27 | S | $-CH(CH_3)-$ | $-COOC_4H_9(s)$ | |
| 2.28 | S | $-CH(CH_3)-$ | $-COOC_4H_9(t)$ | |

Formulierungsbeispiele:

Beispiel F 1 : Formulierungsbeispiele für Wirkstoffe der Formel I (% = Gewichtsprozent)

| a) Spritzpulver | a) | b) | c) |
|---|---|---|---|
| Wirkstoff Nr. 1.02 | 20 % | 50 % | 0,5 % |
| Na-Ligninsulfonat | 5 % | 5 % | 4 % |
| Na-Laurylsulfat | 3 % | - | - |
| Na-Diisobutylnaphthalinsulfonat | - | 6 % | 6 % |
| Octylphenolpolyethylenglykoläther (7-8 Mol AeO) | - | 2 % | 2 % |
| Hochdisperse Kieselsäure | 5 % | 27 % | 27 % |
| Kaolin | 67 % | 10 % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff Nr. 1.02 | 10 % | 1 % |
| Octylphenolpolyethylenglykolether (4-5 Mol AeO) | 3 % | 3 % |
| Ca-Dodecylbenzolsulfonat | 3 % | 3 % |
| Ricinusolpolyglykolether (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff Nr. 1.02 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff Nr. 1.02 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocket.

e) Umhüllungs-Granulat

| | |
|---|---|
| Wirkstoff Nr. 1.02 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff Nr. 1.02 | 40 % | 5 % |
| Ethylenglykol | 10 % | 10 % |
| Nonylphenolpolyethylenglykolether (15 Mol AeO) | 6 % | 1 % |
| Na-Ligninsulfonat | 10 % | 5 % |
| Carboxymethylcellulose | 1 % | 1 % |
| 37%-ige wässrige Formaldehyd-Lösung | 0,2 % | 0,2 % |
| Silikonöl in Form einer 75%-igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff Nr. 1.02 | 5 % |
| Isopropylamin | 1 % |
| Octylphenolpolyethylenglykolether (78 Mol AeO) | 3 % |
| Wasser | 91 % |

Biologische Beispiele

Beispiel B1: Pre-emergente Herbizid-Wirkung

Im Gewächshaus wird unmittelbar nach der Einsaat der Versuchspflanzen in Saatschalen die Erdobefläche mit einer wässrigen Spritzbrühe entsprechend einer Aufwandmenge von 250 g Wirksubstanz/Hektar behandelt. Die Saatschalen werden im Gewächshaus bei 22-25°C und 50-70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich mit einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabellen 1 und 2 starke Herbizidwirkung. Die Ergebnisse der Verbindung der Formel 1.54 sind in Tabelle 3 zusammengefasst:

TABELLE 3

| Testpflanze | Aufwandmenge [g/ha] 250 | 125 |
|---|---|---|
| Weizen | 7 | 9 |
| Mais | 8 | 9 |
| Sorghum | 8 | 9 |
| Reis | 7 | 9 |
| Soja | 9 | 9 |
| Abutilon | 1 | 1 |
| Amaranthus ret. | 1 | 2 |
| Chemopodium | 1 | 1 |
| Solanum n. | 1 | 1 |
| Chrysanthe. leuc. | 1 | 1 |
| Viola tricolor | 1 | 1 |
| Veronica Sp. | 1 | 1 |

Beispiel B2: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 30-1000 g Wirksubstanz pro Hektar auf die Pflanzen gespritzt und diese bei 24°-26°C und 45-60 % relativer Luftfeuchtigkeit gehalten. 15 Tage nach der Behandlung wird die Herbizidwirkung mit einem neunstufigen ( 1 = vollständige Schädigung, 9 = keine Wirkung) Boniturschema im Vergleich mit einer unbehandelten Kontrollgruppe bewertet. Einzelergebnisse sind in den Tabellen 4, 5 und 6 zusammengestellt:

Tabelle 4: Herbizid-Wi. .ng der Verbindung Nr. 1.02:

| Pflanze | Aufwandmenge [g/ha] | | | |
|---|---|---|---|---|
| | 1000 | 500 | 250 | 125 |
| Abutilon | 1 | 1 | 1 | 1 |
| Sida spinosa | 1 | 1 | 1 | 1 |
| Xanthium Sp. | 1 | 1 | 1 | 1 |
| Amaranthus ret. | 1 | 1 | 1 | 1 |
| Chenopodium Sp. | 1 | 1 | 1 | 1 |
| Solanum nigrum | 1 | 1 | 1 | 1 |
| Ipomoea | 1 | 1 | 1 | 1 |
| Sinapis | 1 | 1 | 1 | 1 |
| Stellaria | 1 | 1 | 1 | 1 |
| Chrysanthe. leuc. | 1 | 1 | 1 | 1 |
| Galium aparine | 1 | 1 | 1 | 1 |
| Viola tricolor | 1 | 1 | 1 | 1 |
| Veronica Sp. | 1 | 1 | 1 | 1 |

Tabelle 5: Herbizid-Wirkung der Verbindung Nr. 1.03:

| Pflanze | Aufwandmenge [g/ha] | | | | | |
|---|---|---|---|---|---|---|
| | 1000 | 500 | 250 | 125 | 60 | 30 |
| Abutilon | 1 | 1 | 1 | 1 | 1 | 1 |
| Sida spinosa | 1 | 1 | 1 | 1 | 1 | 2 |
| Amaranthus ret. | 1 | 1 | 1 | 1 | 1 | 3 |
| Solanum nigrum | 1 | 1 | 1 | 2 | 2 | 3 |
| Sinapis | 1 | 1 | 1 | 1 | 1 | 1 |
| Stellaria | 1 | 1 | 1 | 1 | 1 | 2 |
| Viola tricolor | 1 | 1 | 1 | 1 | 1 | 1 |
| Veronica Sp. | 1 | 1 | 1 | 1 | 1 | 1 |

TABELLE 6

Herbizid-Wirkung der Verbindung No. 1.54

| Testpflanze | Aufwandmenge [g/ha] 250 | 125 |
|---|---|---|
| Gerste | 8 | 9 |
| Weizen | 9 | 9 |
| Reis | 9 | 9 |
| Abutilon | 1 | 1 |
| Sida spinosa | 1 | 1 |
| Xanthium sp. | 1 | 1 |
| Chemopodium | 1 | 1 |
| Solanum n. | 1 | 1 |
| Ipomea | 1 | 1 |
| Sinapis | 1 | 1 |
| Chrysanthe. leuc. | 1 | 1 |
| Viola tricolor | 1 | 1 |

Beispiel B3: Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdobefläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefässe. Die verwendete Dosis entspricht einer Wirkstoffmenge von 250 g AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Tabellen 1und 2 schädigen dabei die Unkräuter, nicht aber den Reis.

Beispiel B4: Wuchshemmung bei tropischen Bodenbedecker-Leguminosen (cover crops)

Die Versuchspflanzen Centrosema pubescens und Psophocarpus palustris werden in 4 cm-Torftöpfen mit

Landerde (45 %), Torf (45 %) und Zonolit (10 %) durch Stecklinge vermehrt. Die Anzucht erfolgt im Gewächshaus bei einer Tagestemperatur von 27°C und einer Nachttemperatur von 23°C. Die Beleuchtungsdauer ist mindestens 14 Stunden/Tag bei einer Intensität von mindestens 7000 Lux.

Ca. 50 Tage nach Ansatz der Stecklinge erfolgt das Vertopfen in 13 cm-Töpfe, 4-5 Pflanzen/Topf. Nach weiteren 60 Tagen werden die Pflanzen auf ca. 15 cm Höhe zurückgeschnitten und appliziert. Dabei werden sie mit 0,1 bis 300 g Wirkstoff/ha (in der Regel 25%-ig formuliert) in wässriger Spritzbrühe besprüht. Die Wasseraufwandmenge beträgt ca. 200 l/ha.

4 Wochen nach der Applikation wird der Neuzuwachs im Gewicht ermittelt und in % zum Durchschnitt der unbehandelten Kontrollen dargestellt. Die nekrotischen Schäden sind in % zur gesamten Blattfläche aufgeführt.

Der Neuzuwachs der behandelten Pflanzen erweist sich als deutlich geringer als der der unbehandelten Kontrollen.

Beispiel B5: Wuchsregulierung an Sojabohnen

Versuchspflanzen der Sorte Williams werden in 11 cm-Tontöpfen mit Landerde (45 %), Torf (45 %) und Zeolit (10 %) angesät und in der Klimakammer bei einer Tagestemperatur von 24°C und einer Nachttemperatur von 19°C angezogen. Die Beleuchtungsdauer ist 16 Stunden pro Tage bei einer Intensität von ca. 350 Mikro-Einstein.

Ca. 24 Tage nach der Saat erfolgt das Umtopfen in 18 cm-Töpfe, 2 Pflanzen/Topf. Nach weiteren 12 Tagen und im Stadium von 5-6 Trifolia-Blätter findet die Applikation mit 0,1 bis 300 g Wirkstoff/ha statt, in der Regel 25%-ig formuliert und in wässriger Spritzbrühe. Die Wasseraufwandmenge beträgt ca. 200 l/ha.

Ca. 4 Wochen nach der Applikation findet die Auswertung statt. Hierbei wird die Höhe des Neuzuwachses gemessen und in % zum Durchschnitt der unbehandelten Kontrollen dargestellt. Die nekrotischen Schäden sind in % zur gesamten Blattfläche aufgeführt.

Die behandelten Pflanzen zeigen einen deutlich geringeren Neuzuwachs als die unbehandelten Kontrollen.

Beispiel B6: Wuchshemmung bei Getreide

Versuchspflanzen (Sommergerste der Sorte Iban) werden in 15 cm-Kunststofftöpfen mit steriler Landerde angesät und in der Klimakammer bei einer Tagestemperatur von 10-15°C und einer Nachttemperatur von 5-10°C angezogen. Die Beleuchtungsdauer ist 13.5 Stunden pro Tag bei einer Intensität von ca. 25000 Lux.

Ca. 34 Tage nach der Saat und dem Ausdünnen auf 4 Pflanzen/Topf erfolgt die Applikation mit 0,1 bis 300 g Wirkstoff/ha, in der Regel 25%-ig formuliert und in wässriger Spritzbrühe. Die Wasseraufwandmenge ist ca. 500 l/ha. Nach der Applikation werden die Pflanzen im Gewächshaus bei einer Tagestemperatur von mindestens 10°C aufgestellt. Die Beleuchtungsdauer ist mind. 13.5 Stunden/Tag.

Ca. 28 Tage nach der Behandlung findet die Auswertung statt. Hierbei wird die Höhe des Neuzuwachses in % zum Durchschnitt der unbehandelten Kontrollen dargestellt. Die nekrotischen Schäden sind in % zur gesamten Blattfläche aufgeführt.

Die behandelten Pflanzen weisen im Vergleich zu unbehandelten Kontrollen eine Verringerung des Neuzuwachsese auf.

Beispiel B7: Wuchshemmung bei Gräsern

Eine Mischung von Gräsern (z.B. Poa, Festuca, Lolium, Bromus, Cynosurus) und Klee (Trifolium pratense-/repens) wird in 15 cm-Kunststofftöpfen mit steriler Landerde angesät und im Gewächshaus bei einer Tagestemperatur von 21°C und einer Nachttemperatur von 17°C angezogen. Die Beleuchtungsdauer ist 13.5 Stunden/Tag bei einer Lichtintensität von mind. 7000 Lux. Nach dem Auflauf werden die Pflanzen wöchentlich auf ca. 6 cm Höhe zurückgeschnitten. Ca. 42 Tage nach der Saat und 1 Tag nach dem letzten Schnitt erfolgt die Applikation mit 0,1 bis 300 g Wirkstoff/ha, in der Regel 25%-ig formuliert und in wässriger Spritzbrühe. Die Wasseraufwand- menge ist ca. 500 l/ha.

Ca. 3 Wochen nach der Behandlung findet die Auswertung statt. Hierbei wird die Höhe des Neuzuwachses gemessen und in % zum Durchschnitt der unbehandelten Kontrollen dargestellt. Die nekrotischen Schäden sind in % zur gesamten Blattfläche aufgeführt.

Die geprüften Verbindungen der Tabellen 1 und 2 bewirken eine Reduzierung des Neuzuwachses im Vergleich zur unbehandelten Kontrolle.

**Patentansprüche**

1. Verbindung der Formel I

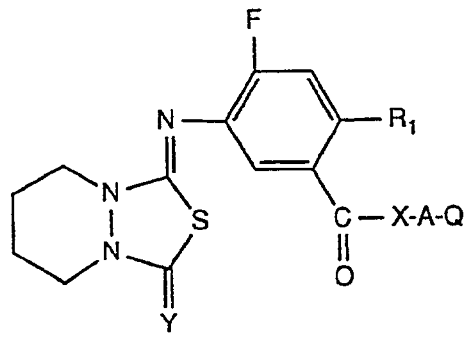

(I)

worin

$R_1$ Halogen;

X Sauerstoff; oder Schwefel;

Y Sauerstoff; oder Schwefel;

A eine geradkettige oder verzweigte $C_1$-$C_4$-Alkylenkette;

Q Hydroxyl; Halogen; Cyano; unsubstituiertes oder durch Cyano oder Halogen substituiertes $C_2$-$C_6$-Alkenyl; $C_2$-$C_4$-Alkinyl; $-CR_2{=}CH\text{-}COOR_3$; $-CH[N(R_2)_2]COOR_2$; $-NR_4(R_5)$; $-CO\text{-}NR_6R_7$; $-COON{=}CR_8(R_8)$; $-C(R_2)(OR_9)_2$; $-Si(R_{10})_3$; $-COOCH_2Si(CH_3)_2$-$C_1$-$C_6$-Alkyl;

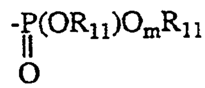 ;

$-CON(R_{12})SO_2$-$C_1$-$C_6$-Alkyl; $-CON(R_{12})SO_2$-$C_1$-$C_4$-Halogenalkyl; $C_1$-$C_6$-Alkylcarbonyl; $C_2$-$C_8$-Alkoxyalkylcarbonyl; Benzoyl; Benzylcarbonyl; $-COOR_{16}$; $-CO\text{-}N(R_2)CH_2\text{-}CH(O\text{-}C_1\text{-}C_6\text{-Alkyl})_2$; $-COO(CH_2)_jN(R_2)_2$; $-S(O)_k\text{-}R_{14}$; $-S(O)_k\text{-}A'\text{-}COOR_{13}$; oder

einen über Kohlenstoff oder Stickstoff gebundenen 5- oder 6-gliedrigen Heterocyclus, welcher 1 bis 3 Heteroatome ausgewählt aus der Gruppe N, O und S enthält, wobei diese Heterocyclen ihrerseits noch benzanelliert sein und bis zu zweifach durch Halogen, $C_1$-$C_4$-Alkyl, $C_1$-$C_3$-Halogenalkyl, $C_1$-$C_3$-Alkoxy, $C_1$-$C_3$-Halogenalkoxy, $C_1$-$C_3$-Dialkylamino, Hydroxy oder Carbonylgruppen substituiert sein können;

$R_2$ Wasserstoff; $C_1$-$C_6$-Alkyl; oder $C_2$-$C_6$Alkoxyalkyl;

$R_3$ $C_1$-$C_6$-Alkyl; oder $C_1$-$C_6$-Hydroxyalkyl;

$R_4$ und $R_5$ unabhängig voneinander je Wasserstoff; $C_1$-$C_6$-Alkyl; $C_2$-$C_6$Alkoxyalkyl; $C_3$-$C_6$-Alkenyl; $C_3$-$C_6$-Alkinyl; $C_3$-$C_6$-Cycloalkyl; 2-Furanylmethyl; 2-Tetrahydrofuranylmethyl; 2-(5-Methyl)-tetrahydrofuranylmethyl; oder 2-Thienylmethyl;

$R_6$ und $R_7$ unabhängig voneinander je Wasserstoff; $C_1$-$C_{12}$-Alkyl; $C_3$-$C_8$-Alkenyl; $C_3$-$C_6$-Alkinyl; $C_2$-$C_8$ Alkoxyalkyl; $C_1$-$C_4$-Alkoxy; Benzyl; Phenyl; oder Cyano-$C_1$-$C_4$-alkyl;

oder

$R_6$ und $R_7$ zusammen mit dem Stickstoffatom, an das sie gebunden sind, einen unsubstituierten oder bis zu zweifach durch $C_1$-$C_4$-Alkyl substituierten Pyrrolidino-, Piperidino-, Morpholino-, Thiomorpholino-, 4-Methylpiperazino-, Pyrazolino-, Imidazolino- oder 1,2,4-Triazolinrest;

$R_8$ unabhängig voneinander je $C_1$-$C_6$-Alkyl; oder zusammen eine $C_3$-$C_7$-Alkylenkette;

$R_9$ unabhängig voneinander $C_1$-$C_4$-Alkyl; oder $C_1$-$C_4$-Haloalkyl; oder zusammen eine Ethano-, Propano- oder Cyclohexan-1,2-diylbrücke;

$R_{10}$ unabhängig voneinander $C_1$-$C_6$-Alkyl; oder $C_1$-$C_6$-Alkoxy;

$R_{11}$ unabhängig voneinander Wasserstoff; $C_1$-$C_6$-Alkyl; $C_1$-$C_6$-Haloalkyl;

Cyano-$C_1$-$C_6$-alkyl; $C_3$-$C_4$-Alkenyl; oder $C_3$-$C_4$-Alkinyl;

$R_{12}$ $C_1$-$C_4$-Alkyl; oder $C_3$-$C_7$-Cycloalkyl;

A' eine geradkettige oder verzweigte $C_1$-$C_4$-Alkylenkette;

$R_{13}$ Wasserstoff; $C_1$-$C_{12}$-Alkyl; $C_3$-$C_7$-Alkenyl; $C_3$-$C_6$-Alkinyl; $C_3$-$C_7$-Cycloalkyl; $C_2$-$C_8$-Alkoxyalkyl; $C_3$-$C_5$-Alkenyloxy-$C_1$-$C_4$-alkyl; $C_1$-$C_4$-Thioalkyl-$C_1$-$C_4$-alkyl; oder $C_1$-$C_4$-Dialkylamino-$C_1$-$C_4$-alkyl;

$R_{14}$ $C_1$-$C_{10}$-Alk

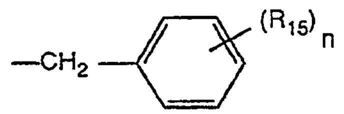 ; oder 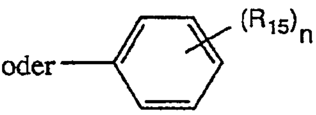 ;

$R_{15}$ $C_1$-$C_4$-Alkyl; Halogen; oder $C_1$-$C_4$-Alkoxy;

$R_{16}$ Wasserstoff; $C_3$-$C_7$-Alkenyl; $C_3$-$C_6$-Alkinyl; $C_3$-$C_7$-Cycloalkyl; $C_2$-$C_8$-Alkoxyalkyl; $C_3$-$C_5$-Alkenyloxy-$C_1$-$C_4$-alkyl; $C_2$-$C_8$-Alkylthioalkyl; $C_1$-$C_4$-Dialkylamino-$C_1$-$C_4$-alkyl; oder, wenn $R_1$ für Chlor und Y für Sauerstoff steht, $C_1$-$C_{10}$-Alkyl;

j 0; 2; oder 3;

k 0; 1; oder 2;

m 0; oder 1; und

n 0; 1; 2; oder 3;

bedeutet, mit der Massgabe, dass A nicht für Methano steht wenn X Sauerstoff und Q Hydroxyl oder Halogen bedeutet.

**2.** Verbindung der Formel I gemäss Anspruch 1, worin

$R_1$ Fluor, Chlor; oder Brom;

A eine geradkettige oder verzweigte $C_1$-$C_4$-Alkylenkette;

n 0; 1; oder 2;

Q $C_1$-$C_{10}$-Alkylthio; $COOR_{16}$; $-S(O)_kR_{14}$; $-NR_4(R_5)$; einen gegebenenfalls bis zu zweifach durch $C_1$-$C_4$-Alkyl oder einfach durch eine Carbonylgruppe substituierten heterocyclischen Rest ausgewählt aus der Gruppe Pyridinyl, 1,3-Thiazolyl, Thiophenyl, Pyrrolidinyl, Morpholinyl, Furanyl, Indolyl, Pyrazolyl, 1,2-Oxazolyl, Pyrrolyl, Imidazolyl, Pyrazolyl und 1,2,4-Triazolyl;

$R_4$ und $R_5$ unabhängig voneinander $C_1$-$C_4$-Alkyl;

$R_{14}$ 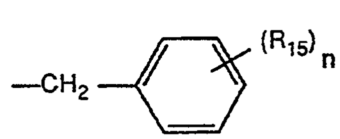 ; oder 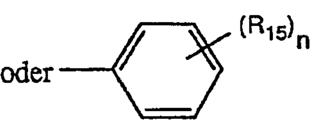 ;

k 0; 1; oder 2;

n 0; 1 ; 2; oder 3;

$R_{15}$ $C_1$-$C_4$-Alkyl; Fluor, Chlor, Brom; oder $C_1$-$C_4$-Alkoxy; und

$R_{16}$ Cyclohexyl; $C_1$-$C_4$-Alkoxy-$C_1$-$C_4$-alkyl; $C_1$-$C_4$-Alkylthio-$C_1$-$C_4$-alkyl; $C_1$-$C_4$-Dialkylamino-$C_1$-$C_4$-alkyl; oder, wenn $R_1$ für Chlor und Y für Sauerstoff steht, $C_1$-$C_{10}$-Alkyl;

bedeutet.

**3.** Verbindung der Formel Ia, gemäss Anspruch 1,

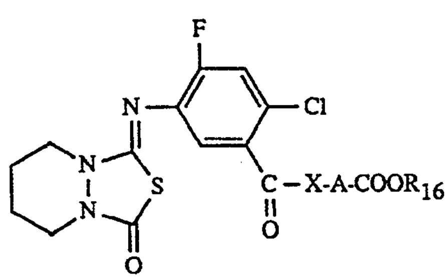

(Ia)

worin

X Sauerstoff oder Schwefel;

A eine geradkettige oder verzweigte $C_1$-$C_4$-Alkylenkette und

$R_{16}$ $C_1$-$C_{10}$-Alkyl bedeutet.

4. Verbindung der Formel Ib, gemäss Anspruch 1,

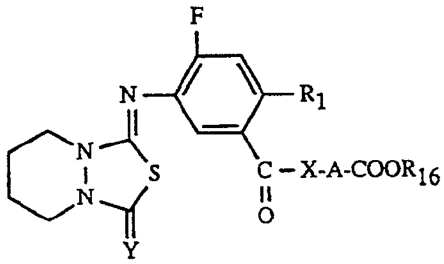

(Ib),

worin

Y Sauerstoff;

X Sauerstoff oder Schwefel;

A eine $C_1$-$C_2$-Alkylenkette; und

$R_{16}$ $C_1$-$C_4$-Alkoxy-$C_1$-$C_4$-alkyl; oder $C_1$-$C_4$-Alkylthio-$C_1$-$C_4$-alkyl; bedeutet.

5. Verbindung nach Anspruch 1 der Formel Ic,

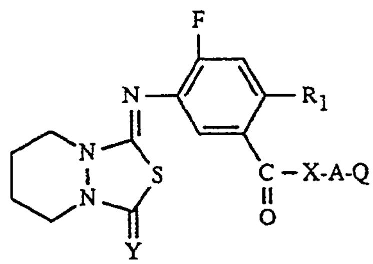

(Ic),

worin

Y Sauerstoff;

X Sauerstoff; oder Schwefel;

$R_1$ Chlor;

A eine $C_1$-$C_2$-Alkylenbrücke; und

Q einen über Kohlenstoff oder Stickstoff gebundenen 5- oder 6-gliedrigen Heterocyclus, welcher 1 bis 3 Heteroatome ausgewählt aus der Gruppe N, O und S enthält, wobei diese Heterocyclen ihrerseits noch benzanelliert sein und bis zu zweifach durch Halogen, $C_1$-$C_4$-Alkyl, $C_1$-$C_3$-Halogenalkyl, $C_1$-$C_3$-Alkoxy, $C_1$-$C_3$-Halogenalkoxy, $C_1$-$C_3$-Dialkylamino, Hydroxy oder Carbonylgruppen substituiert sein können bedeutet.

6. Verbindung gemäss Anspruch 1 der Formel Id,

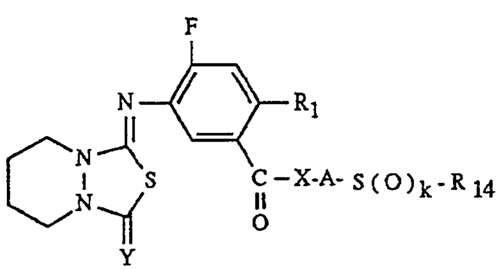

(Id),

worin

Y Sauerstoff;

X Sauerstoff; oder Schwefel;

$R_1$ Chlor;

A eine $C_2$-$C_4$-Alkylenbrücke;

k 0;
$R_{14}$ $C_1$-$C_{10}$-Alkyl;

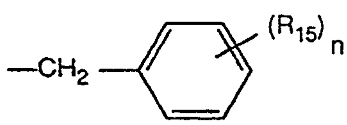 ; oder 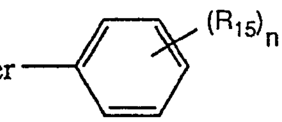 ; und

$R_{15}$ $C_1$-$C_4$-Alkyl; Halogen; oder $C_1$-$C_4$-Alkoxy;
bedeutet.

**7.** Verbindung gemäss einem der Ansprüche 3 bis 6 ausgewählt aus der Gruppe
9-[4-Chlor-2-fluor-5-(methoxycarbonylmethylthiocarbonyl)-phenylimino]-8-thia-1,6-diazabicyclo[4.3.0]nonan-7-on,
9-[4-Chlor-2-fluor-5-(1-methoxycarbonyl-ethylthiocarbonyl)-phenylimino]-8-thia-1,6-diazabicyclo[4.3.0]nonan-7-on,
9-[4-Chlor-2-fluor-5-(methoxycarbonylmethylthiocarbonyl)-phenylimino]-8-thia-1,6-diazabicyclo[4.3.0]nonan-7-on,
9-[4-Chlor-2-fluor-5-(methoxycarbonylmethoxycarbonyl)-phenylimino]-8-thia-1,6-diazabicyclo[4.3.0]nonan-7-on,
9-[4-Chlor-2-fluor-5-(methoxycarbonyl-ethoxycarbonylmethylthiocarbonyl)-phenylimino]-8-thia1,6-diazabicyclo[4.3.0]nonan-7-on,
9-[4-Chlor-2-fluor-5-(2-methoxy-1-methyl-ethoxycarbonylmethylthiocarbonyl)-phenylimino]-8-thia-1,6-diazabicyclo[4.3.0]nonan-7-on,
9-[4-Chlor-2-fluor-5-(2-methoxy-1-methyl-ethoxycarbonyl-1-ethylthiocarbonyl)-phenylimino]-8-thia-1,6-diazabicyclo[4.3.0]nonan-7-on,
9-[4-Chlor-2-fluor-5-(2-methoxy-ethoxycarbonyl-1-ethylthiocarbonyl)-phenylimino]-8-thia1,6-diazabicyclo[4.3.0]nonan-7-on,
9-[4-Chlor-2-fluor-5-(2-ethoxy-ethoxycarbonylmethylthiocarbonyl)-phenylimino]-8-thia-1,6-diazabicyclo[4.3.0]nonan-7-on,
9-{4-Chlor-2-fluor-5-[2-(4-methyl-thiazol-5-yl)-ethoxyarbonyl]-phenylimino}-8-thia-1,6-diazabicyclo[4.3.0]nonan-7-on,
9-{4-Chlor-2-fluor-5-[2-(pyridin-2-yl)ethoxycarbonyl]-phenylimino}-8-thia1,6-diazabicyclo[4.3.0]nonan-7-on,
9-[4-Chlor-2-fluor-5-(2-methylthio-1-methylethoxyarbonyl)-phenylimino]-8-thia-1,6-diazabicyclo[4.3.0]nonan-7-on,
9-[4-Chlor-2-fluor-5-(2-ethylthio-1-methylethoxyarbonyl)-phenylimino]-8-thia1,6-diazabicyclo[4.3.0]nonan-7-on,
9-[4-Chlor-2-fluor-5-(2-propylthio-1-methylethoxyarbonyl)-phenylimino]-8-thia1,6-diazabicyclo[4.3.0]nonan-7-on,
9-[4-Chlor-2-fluor-5-(2-i-propylthio-1-methylethoxyarbonyl)-phenylimino]-8-thia1,6-diazabicyclo[4.3.0]nonan-7-on,
9-[4-Chlor-2-fluor-5-(2-n-butylthio-1-methylethoxyarbonyl)-phenylimino]-8-thia1,6-diazabicyclo[4.3.0]nonan-7-on,
9-[4-Chlor-2-fluor-5-(2-phenylthio-ethoxyarbonyl)-phenylimino]-8-thia1,6-diazabicyclo[4.3.0]nonan-7-on und
9-[4-Chlor-2-fluor-5-(2-p-chlorphenylthio-1-methyl-ethoxyarbonyl)-phenylimino]-8-thia1,6-diazabicyclo[4.3.0]nonan-7-on.

**8.** Verfahren zur Herstellung einer Verbindung der Formel I gemäss Anspruch 1, dadurch gekennzeichnet, dass man
a) einen Isothiocyanato-benzoesäureester der Formel II

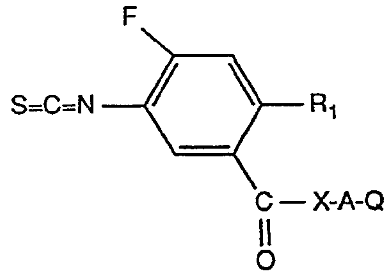

(II),

worin $R_1$, X, A und Q die unter der Formel I angegebene Bedeutung haben, mit Hexahydropyridazin in die Verbindung der Formel III

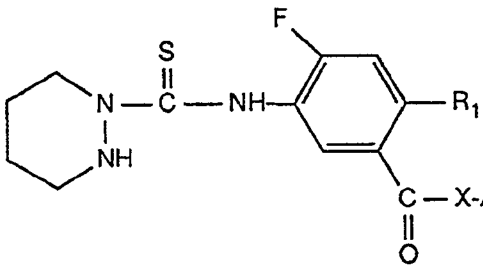

(III)

überführt und diese anschliessend mit einer Verbindung der Formel IV

$CYCl_2$ (IV),

worin Y für Sauerstoff oder Schwefel steht, in Gegenwart einer Base umsetzt, oder

b) eine Verbindung der Formel VI

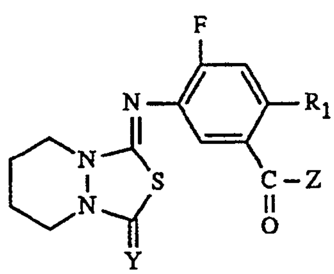

(VI),

worin $R_1$ und Y wie unter Formel I definiert sind und Z für eine nucleofuge Gruppe, wie Chlor, Brom, Azido oder -O-CO-$C_1$-$C_4$-Alkyl oder für OH steht, mit einem Alkohol oder Thiol der Formel VII

H - X - A - Q (VII)

worin X, A und Q wie unter Formel I definiert sind, umsetzt oder

c) ein Carbonsäurederivat der Formel VIII

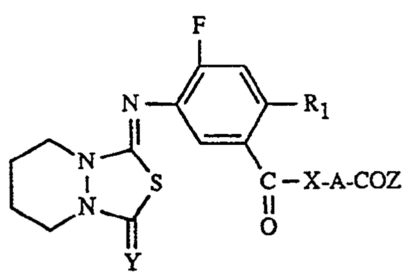

(VIII),

worin die Reste X, Y, $R_1$ und A wie unter Formel I definiert sind und Z für eine nucleofuge Gruppe, wie Chlor, Brom, Azido oder -O-CO-$C_1$-$C_4$-Alkyl oder für OH steht, mit einem Alkohol oder Thiol der Formel IX

HO - $R_{16}$ (IX)

worin $R_{16}$ wie unter Formel I definiert sind. zu einer Verbindung der Formel I'

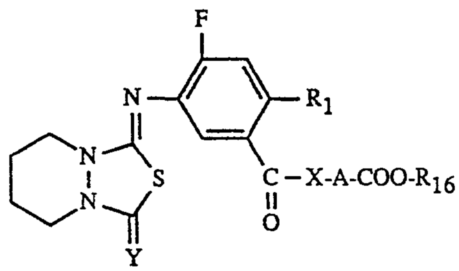

(I')

worin die Reste X, Y, $R_1$, $R_{16}$ und A wie unter Formel I definiert sind, umsetzt oder d) einen Thioether der Formel If

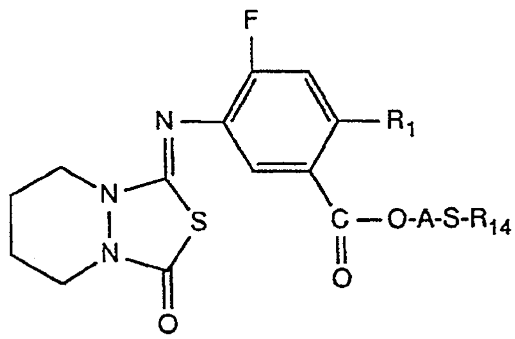

(If),

worin $R_1$, $R_{14}$ und A die unter Formel I in Anspruch 1 angegebene Bedeutung haben, zu einer Verbindung der Formel Ie

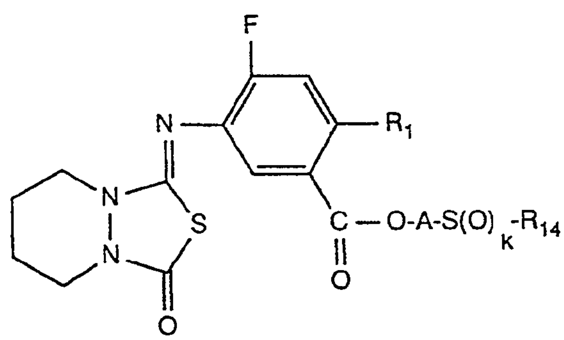

(Ie),

oxidiert.

**9.** Isothiocyanato-benzoesäureester der Formel II

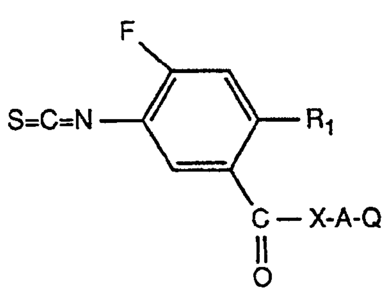

(II),

worin $R_1$, X, A und Q wie in Anspruch 1 definiert sind.

**10.** Thioharnstoff der Formel III

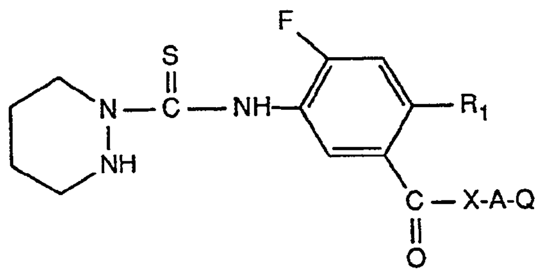
(III),

worin $R_1$, X, A und Q wie in Anspruch 1 definiert sind.

**11.** Verfahren zur Herstellung eines Isothiocyanato-benzoesäureester der Formel II

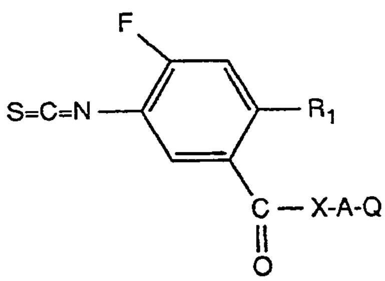
(II),

nach Anspruch 9, dadurch gekennzeichnet, dass man ein Anilin der Formel X

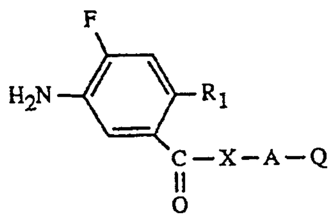
(X),

worin $R_1$, X, A und Q wie zuvor definiert sind, mit Thiophosgen umsetzt.

**12.** Herbizides Mittel gekennzeichnet durch einen Gehalt an einem oder mehreren Wirkstoffen der Formel I gemäss einem der Ansprüche 1 bis 7.

**13.** Verfahren zur Bekämpfung unerwünschten Pflanzenwachstums, dadurch gekennzeichnet, dass man einen Wirkstoff der Formel I, gemäss einem der Ansprüche 1 bis 7 der ein diesen Wirkstoff enthaltendes Mittel in einer wirksamen Menge auf die Pflanzen oder deren Lebensraum appliziert.

**14.** Pflanzenwuchsregulatorisches Mittel gekennzeichnet durch einen Gehalt an einem oder mehreren Wirkstoffen der Formel I gemäss einem der Ansprüche 1 bis 7.

**15.** Verfahren zur Beeinflussung des Pflanzenwachstums zum Zweck der Ertragssteigerung, dadurch gekennzeichnet, dass man einen Wirkstoff der Formel I, gemäss einem der Ansprüche 1 bis 7, oder ein diesen Wirkstoff enthaltendes Mittel in einer wirksamen Menge auf die Pflanzen oder deren Lebensraum appliziert.

**Patentansprüche für folgenden Vertragsstaat: ES**

1. Verfahren zur Herstellung einer Verbindung der Formel I

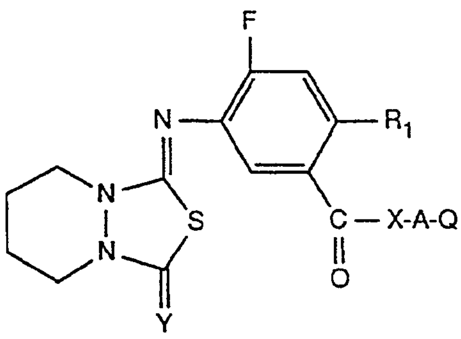

(I)

worin

$R_1$ Halogen;
X Sauerstoff; oder Schwefel;
Y Sauerstoff; oder Schwefel;
A eine geradkettige oder verzweigte $C_1$-$C_4$-Alkylenkette;
Q Hydroxyl; Halogen; Cyano; unsubstituiertes oder durch Cyano oder Halogen substituiertes $C_2$-$C_6$-Alkenyl; $C_2$-$C_4$-Alkinyl; $-CR_2=CH-COOR_3$; $-CH[N(R_2)_2]COOR_2$; $-NR_4(R_5)$; $-CO-NR_6R_7$; $-COON=CR_8(R_8)$; $-C(R_2)(OR_9)_2$; $-Si(R_{10})_3$; $-COOCH_2Si(CH_3)_2$-$C_1$-$C_6$-Alkyl;

$$-\underset{\underset{O}{\|}}{P}(OR_{11})O_mR_{11}\ ;$$

$-CON(R_{12})SO_2$-$C_1$-$C_6$-Alkyl; $-CON(R_{12})SO_2$-$C_1$-$C_4$-Halogenalkyl; $C_1$-$C_6$-Alkylcarbonyl; $C_2$-$C_6$-Alkoxyalkylcarbonyl; Benzoyl; Benzylcarbonyl; $-COOR_{16}$; $-CO-N(R_2)CH_2-CH(O$-$C_1$-$C_6$-Alkyl$)_2$; $-COO(CH_2)_jN(R_2)_2$; $-S(O)_k-R_{14}$; $-S(O)_k-A'-COOR_{13}$; oder
einen über Kohlenstoff oder Stickstoff gebundenen 5- oder 6-gliedrigen Heterocyclus, welcher 1 bis 3 Heteroatome ausgewählt aus der Gruppe N, O und S enthält, wobei diese Heterocyclen ihrerseits noch benzanelliert sein und bis zu zweifach durch Halogen, $C_1$-$C_4$-Alkyl, $C_1$-$C_3$-Halogenalkyl, $C_1$-$C_3$-Alkoxy, $C_1$-$C_3$-Halogenalkoxy, $C_1$-$C_3$-Dialkylamino, Hydroxy oder Carbonylgruppen substituiert sein können;
$R_2$ Wasserstoff; $C_1$-$C_6$-Alkyl; oder $C_2$-$C_6$Alkoxyalkyl;
$R_3$ $C_1$-$C_6$-Alkyl; oder $C_1$-$C_6$-Hydroxyalkyl;
$R_4$ und $R_5$ unabhängig voneinander je Wasserstoff; $C_1$-$C_6$-Alkyl; $C_2$-$C_6$Alkoxyalkyl; $C_3$-$C_6$-Alkenyl; $C_3$-$C_3$-Alkinyl; $C_3$-$C_6$-Cycloalkyl; 2-Furanylmethyl; 2-Tetrahydrofuranylmethyl; 2-(5-Methyl)-tetrahydrofuranylmethyl; oder 2-Thienylmethyl;
$R_6$ und $R_7$ unabhängig voneinander je Wasserstoff; $C_1$-$C_{12}$-Alkyl; $C_3$-$C_8$-Alkenyl; $C_3$-$C_6$-Alkinyl; $C_2$-$C_8$Alkoxyalkyl; $C_1$-$C_4$-Alkoxy; Benzyl; Phenyl; oder Cyano-$C_1$-$C_4$-alkyl;
oder
$R_6$ und $R_7$ zusammen mit dem Stickstoffatom, an das sie gebunden sind, einen unsubstituierten oder bis zu zweifach durch $C_1$-$C_4$-Alkyl substituierten Pyrrolidino-, Piperidino-, Morpholino-, Thiomorpholino-, 4-Methylpiperazino-, Pyrazolino-, Imidazolino- oder 1,2,4-Triazolinrest;
$R_8$ unabhängig voneinander je $C_1$-$C_6$-Alkyl; oder zusammen eine $C_3$-$C_7$-Alkylenkette;
$R_9$ unabhängig voneinander $C_1$-$C_4$-Alkyl; oder $C_1$-$C_4$-Haloalkyl; oder zusammen eine Ethano-, Propano- oder Cyclohexan-1,2-diylbrücke;
$R_{10}$ unabhängig voneinander $C_1$-$C_6$-Alkyl; oder $C_1$-$C_6$-Alkoxy;
$R_{11}$ unabhängig voneinander Wasserstoff;
$C_1$-$C_6$-Alkyl; $C_1$-$C_6$-Haloalkyl; Cyano-$C_1$-$C_6$-alkyl; $C_3$-$C_4$-Alkenyl; oder $C_3$-$C_4$-Alkinyl;
$R_{12}$ $C_1$-$C_4$-Alkyl; oder $C_3$-$C_7$-Cycloalkyl;
A' eine geradkettige oder verzweigte $C_1$-$C_4$-Alkylenkette;
$R_{13}$ Wasserstoff; $C_1$-$C_{12}$-Alkyl; $C_3$-$C_7$-Alkenyl; $C_3$-$C_6$-Alkinyl; $C_3$-$C_7$-Cycloalkyl;
$C_2$-$C_8$-Alkoxyalkyl; $C_3$-$C_5$-Alkenyloxy-$C_1$-$C_4$-alkyl; $C_1$-$C_4$-Thioalyl-$C_1$-$C_4$-alkyl;
oder $C_1$-$C_4$-Dialkylamino-$C_1$-$C_4$-alkyl;

$R_{14}$ $C_1$-$C_{10}$-Alkyl;

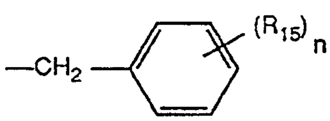 ; oder 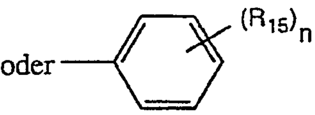 ;

$R_{15}$ $C_1$-$C_4$-Alkyl; Halogen; oder $C_1$-$C_4$-Alkoxy;

$R_{16}$ Wasserstoff; $C_3$-$C_7$-Alkenyl; $C_3$-$C_6$-Alkinyl; $C_3$-$C_7$-Cycloalkyl; $C_2$-$C_8$-Alkoxyalkyl; $C_3$-$C_5$-Alkenyloxy-$C_1$-$C_4$-alkyl; $C_2$-$C_8$-Alkylthioalkyl; $C_1$-$C_4$-Dialkylamino-$C_1$-$C_4$-alkyl; oder, wenn $R_1$ für Chlor und Y für Sauerstoff steht, $C_1$-$C_{10}$-Alkyl;

j 0; 2; oder 3;

k 0; 1; oder 2;

m 0; oder 1; und

n 0; 1; 2; oder 3;

bedeutet, mit der Massgabe, dass A nicht für Methano steht wenn X Sauerstoff und Q Hydroxyl oder Halogen bedeutet, dadurch gekennzeichnet, dass man

a) einen Isothiocyanato-benzoesäureester der Formel II

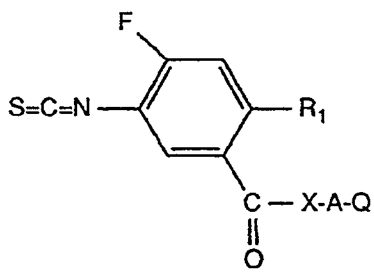 (II),

worin $R_1$, X, A und Q die unter der Formel I angegebene Bedeutung haben, mit Hexahydropyridazin in die Verbindung der Formel III

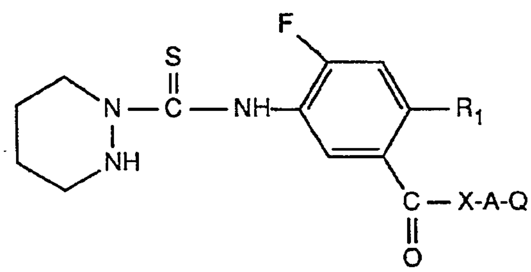 (III)

überführt und diese anschliessend mit einer Verbindung der Formel IV

$CYCl_2$ (IV),

worin Y für Sauerstoff oder Schwefel steht, in Gegenwart einer Base umsetzt, oder

b) eine Verbindung der Formel VI

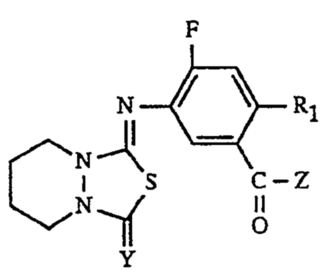 (VI),

worin $R_1$ und Y wie unter Formel I definiert sind und Z für eine nucleofuge Gruppe, wie Chlor, Brom, Azido oder -O-CO-$C_1$-$C_4$-Alkyl oder für OH steht, mit einem Alkohol oder Thiol der Formel VII

H - X - A - Q (VII)

worin X, A und Q wie unter Formel I definiert sind, umsetzt oder

c) ein Carbonsäurederivat der Formel VIII

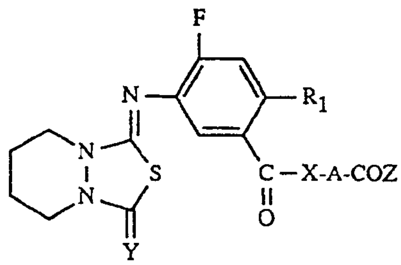

(VIII),

worin die Reste X, Y, $R_1$ und A wie unter Formel I definiert sind und Z für eine nucleofuge Gruppe, wie Chlor, Brom, Azido oder -O-CO-$C_1$-$C_4$-Alkyl oder für OH steht, mit einem Alkohol oder Thiol der Formel IX

HO - $R_{16}$ (IX)

worin $R_{16}$ wie unter Formel I definiert sind.

zu einer Verbindung der Formel I'

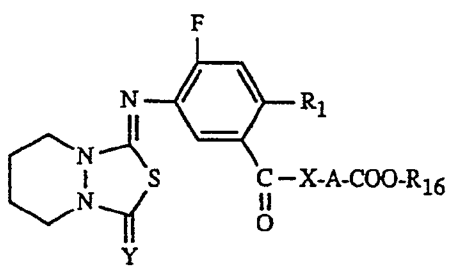

(I')

worin die Reste X, Y, $R_1$, $R_{16}$ und A wie unter Formel I definiert sind, umsetzt oder

d) einen Thioether der Formel If

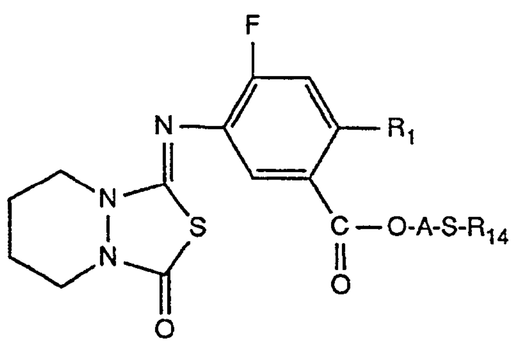

(If),

worin $R_1$, $R_{14}$ und A die unter Formel I in Anspruch 1 angegebene Bedeutung haben, zu einer Verbindung der Formel Ie

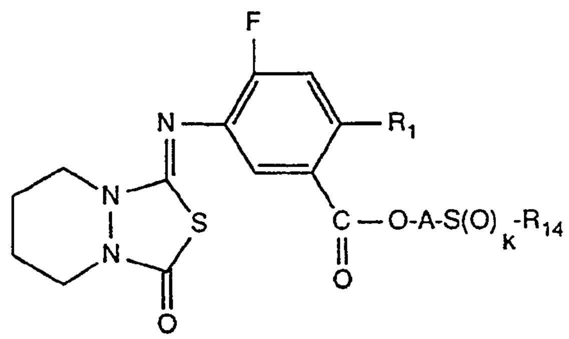

(Ie),

oxidiert.

2. Verfahren nach Anspruch 1 zur Herstellung einer Verbindung der Formel I, worin
   $R_1$ Fluor, Chlor, oder Brom;
   A eine geradkettige oder verzweigte $C_1$-$C_4$-Alkylenkette;
   n 0; 1; oder 2;
   Q $C_1$-$C_{10}$-Alkylthio; $COOR_{16}$; $-S(O)_kR_{14}$; $-NR_4(R_5)$; einen gegebenenfalls bis zu zweifach durch $C_1$-$C_4$-Alkyl oder einfach durch eine Carbonylgruppe substituierten heterocyclischen Rest ausgewählt aus der Gruppe Pyridinyl, 1,3-Thiazolyl, Thiophenyl, Pyrrolidinyl, Morpholinyl, Furanyl, Indolyl, Pyrazolyl, 1,2-Oxazolyl, Pyrrolyl, Imidazolyl, Pyrazolyl und 1,2,4-Triazolyl;
   $R_4$ und $R_5$ unabhängig voneinander $C_1$-$C_4$-Alkyl;

$R_{14}$ 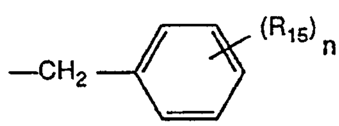 ; oder 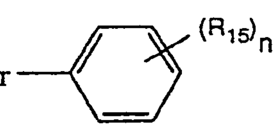 ;

   k 0; 1; oder 2;
   n 0; 1; 2; oder 3;
   $R_{15}$ $C_1$-$C_4$-Alkyl; Fluor, Chlor, Brom; oder $C_1$-$C_4$-Alkoxy; und
   $R_{16}$ Cyclohexyl; $C_1$-$C_4$-Alkoxy-$C_1$-$C_4$-alkyl; $C_1$-$C_4$-Alkylthio-$C_1$-$C_4$-alkyl; $C_1$-$C_4$-Dialkylamino-$C_1$-$C_4$-alkyl; oder, wenn $R_1$ für Chlor und Y für Sauerstoff steht, $C_1$-$C_{10}$-Alkyl;
   bedeutet.

3. Verfahren nach Anspruch 1 zur Herstellung einer Verbindung der Formel Ia,

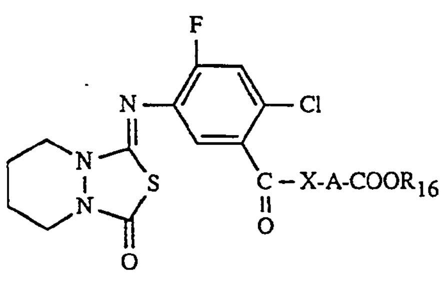

(Ia)

   worin
   X Sauerstoff oder Schwefel;
   A eine geradkettige oder verzweigte $C_1$-$C_4$-Alkylenkette und
   $R_{16}$ $C_1$-$C_{10}$-Alkyl bedeutet.

4. Verfahren nach Anspruch 1 zur Herstellung einer Verbindung der Formel Ib,

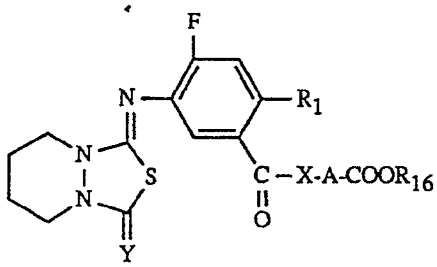

(Ib),

worin

Y Sauerstoff;
X Sauerstoff oder Schwefel;
A eine $C_1$-$C_2$-Alkylenkette; und
$R_{16}$ $C_1$-$C_4$-Alkoxy-$C_1$-$C_4$-alkyl; oder $C_1$-$C_4$-Alkylthio-$C_1$-$C_4$-alkyl; bedeutet.

5. Verfahren nach Anspruch 1 zur Herstellung einer Verbindung der Formel Ic,

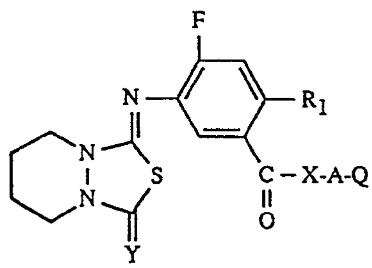

(Ic),

worin

Y Sauerstoff;
X Sauerstoff; oder Schwefel;
$R_1$ Chlor,
A eine $C_1$-$C_2$-Alkylenbrücke; und
Q einen über kohlenstoff oder Stickstoff gebundenen 5- oder 6-gliedrigen Heterocyclus, welcher 1 bis 3 Heteroatome ausgewählt aus der Gruppe N, O und S enthält, wobei diese Heterocyclen ihrerseits noch benzanelliert sein und bis zu zweifach durch Halogen, $C_1$-$C_4$-Alkyl, $C_1$-$C_3$-Halogenalkyl, $C_1$-$C_3$-Alkoxy, $C_1$-$C_3$-Halogenalkoxy, $C_1$-$C_3$-Dialkylamino, Hydroxy oder Carbonylgruppen substituiert sein können bedeutet.

6. Verfahren nach Anspruch 1 zur Herstellung einer Verbindung der Formel Id,

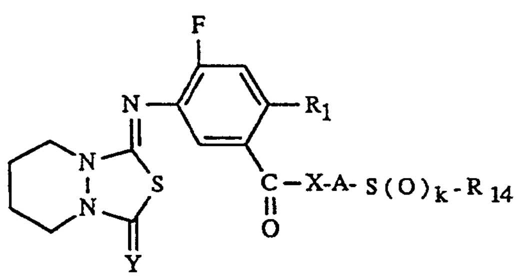

(Id),

worin

Y Sauerstoff;
X Sauerstoff; oder Schwefel;
$R_1$ Chlor;
A eine $C_1$-$C_4$-Alkylenbrücke;
k 0;
$R_{14}$ $C_1$-$C_{10}$-Alkyl;

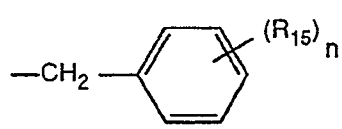 ; oder 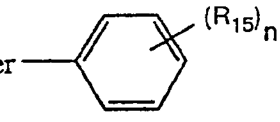 ;

und

$R_{15}$ $C_1$-$C_4$-Alkyl; Halogen; oder $C_1$-$C_4$-Alkoxy;

bedeutet.

**7.** Verfahren nach Anspruch 1 zur Herstellung einer Verbindung ausgewählt aus der Gruppe

9-[4-Chlor-2-fluor-5-(methoxycarbonylmethylthiocarbonyl)-phenylimino]-8-thia-1,6-diazabicyclo [4.3.0]nonan-7-on,

9-[4-Chlor-2-fluor-5-(1-methoxycarbonyl-ethylthiocarbonyl)-phenylimino]-8-thia-1,6-diazabicyclo [4.3.0]nonan-7-on,

9-[4-Chlor-2-fluor-5-(methoxycarbonylmethylthiocarbonyl)-phenylimino]-8-thia-1,6-diazabicyclo [4.3.0]nonan-7-on,

9-[4-Chlor-2-fluor-5-(methoxycarbonylmethoxycarbonyl)-phenylimino]-8-thia-1,6-diazabicyclo [4.3.0]nonan-7-on,

9-[4-Chlor-2-fluor-5-(2-methoxy-ethoxycarbonylmethylthiocarbonyl)-phenylimino]-8-thia1,6-diazabicyclo[4.3.0]nonan-7-on,

9-[4-Chlor-2-fluor-5-(2-methoxy-1-methyl-ethoxycarbonylmethylthiocarbonyl)-phenylimino]-8-thia1,6-diazabicyclo[4.3.0]nonan-7-on,

9-[4-Chlor-2-fluor-5-(2-methoxy-1-methyl-ethoxycarbonyl-1-ethylthiocarbonyl)-phenylimino]-8-thia1,6-diazabicyclo[4.3.0]nonan-7-on,

9-[4-Chlor-2-fluor-5-(2-methoxy-ethoxycarbonyl-1-ethylthiocarbonyl)-phenylimino]-8-thia1,6-diazabicyclo[4.3.0]nonan-7-on,

9-[4-Chlor-2-fluor-5-(2-ethoxy-ethoxycarbonylmet hylthiocarbonyl)-phenylimino]-8-thia1,6-diazabicyclo[4.3.0]nonan-7-on,

9-{4-Chlor-2-fluor-5-[2-(4-methyl-thiazol-5-yl)-ethoxyarbonyl]-phenylimino}-8-thia1,6-diazabicyclo [4.3.0]nonan-7-on,

9-{4-Chlor-2-fluor-5-[2-(pyridin-2-yl)-ethoxyarbonyl]-phenylimino}-8-thia1,6-diazabicyclo[4.3.0] nonan-7-on,

9-[4-Chlor-2-fluor-5-(2-methylthio-1-methylethoxyarbonyl)-phenylimino]-8-thia1,6-diazabicyclo [4.3.0]nonan-7-on,

9-[4-Chlor-2-fluor-5-(2-ethylthio-1-methylethoxyarbonyl)-phenylimino]-8-thia1,6-diazabicyclo[4.3.0] nonan-7-on,

9-[4-Chlor-2-fluor-5-(2-propylthio-1-methylethoxyarbonyl)-phenylimino]-8-thia1,6-diazabicyclo[4.3.0] nonan-7-on,

9-[4-Chlor-2-fluor-5-(2-i-propylthio-1-methylethoxyarbonyl)-phenylimino]-8-thia1,6-diazabicyclo [4.3.0]nonan-7-on,

9-[4-Chlor-2-fluor-5-(2-n-butylthio-1-methylethoxyarbonyl)-phenylimino]-8-thia1,6-diazabicyclo [4.3.0]nonan-7-on,

9-[4-Chlor-2-fluor-5-(2-phenylthio-ethoxyarbonyl)-phenylimino]-8-thia1,6-diazabicyclo[4.3.0]nonan-7-on und

9-[4-Chlor-2-fluor-5-(2-p-chlorphenylthio-1-methyl-ethoxyarbonyl)-phenylimino]-8-thia-1,6-diazabicyclo[4.3.0]nonan-7-on.

**8.** Verfahren zur Herstellung Herstellung eines Isothiocyanato-benzoesäureesters der Formel II

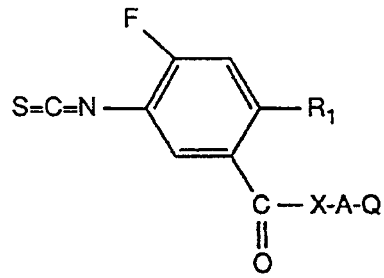

(II),

worin die Reste X, A, Q und $R_1$ wie in einem der Ansprüche 1 bis 7 definiert sind, dadurch gekennzeichnet, dass man ein Anilin der Formel X

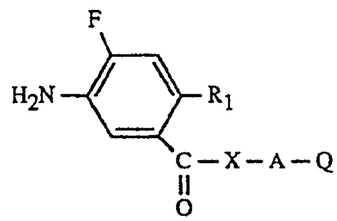

(X),

worin $R_1$, X, A und Q wie zuvor definiert sind, mit Thiophosgen umsetzt.

9. Verfahren zur Herstellung eines herbiziden und/oder pflanzenwuchsregulatorischen Mittels, dadurch gekennzeichnet, dass man eine Verbindung der Formel I,

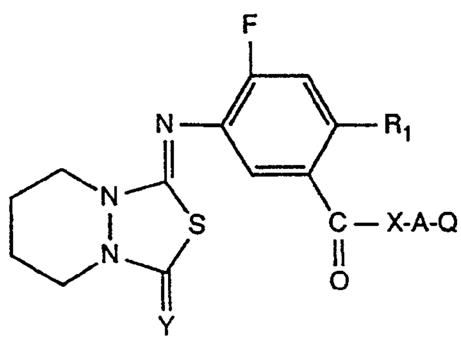

(I)

worin die Reste Y, X, A, Q und $R_1$ wie ine einem der Ansprüche 1 bis 7 definert sind, mit Hilfs- und/oder Trägerstoffen innig vermischt.

10. Verfahren zur Bekämpfung unerwünschten Pflanzenwachstums, dadurch gekennzeichnet, dass man einen Wirkstoff der Formel I,

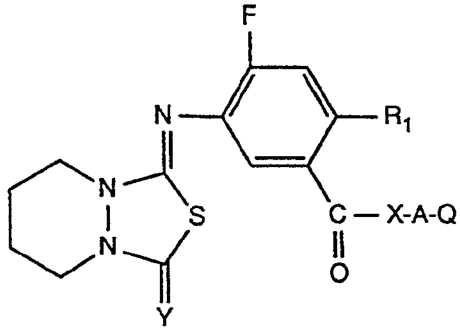

(I)

worin die Reste Y, X, A, Q und $R_1$ wie ine einem der Ansprüche 1 bis 7 definert sind, oder ein diesen Wirkstoff enthaltendes Mittel in einer wirksamen Menge auf die Pflanzen oder deren Lebensraum appliziert.

**11.** Verfahren zur Beeinflussung des Pflanzenwachstums zum Zweck der Eraagssteigerung, dadurch gekennzeichnet, dass man einen Wirkstoff der Formel I,

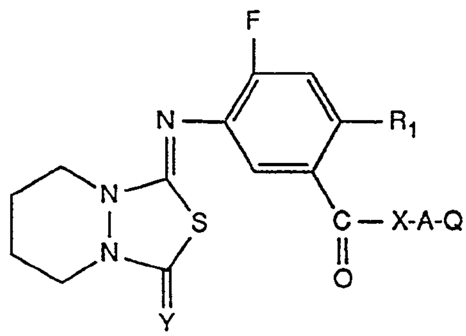

(I)

worin die Reste Y, X, A, Q und $R_1$ wie ine einem der Ansprüche 1 bis 7 definert sind, oder ein diesen Wirkstoff enthaltendes Mittel in einer wirksamen Menge auf die Pflanzen oder deren Lebensraum appliziert.

Europäisches Patentamt

# EUROPÄISCHER RECHERCHENBERICHT

Nummer der Anmeldung

EP 91 81 0168

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, Band 107, Nr. 15, Oktober 1987, Seite 725, Zusammenfassung Nr. 134316u; Columbus, Ohio, US; & JP-A-62 00 091 (KUMIAI CHEMICAL IND. CO., LTD) 06-01-1987<br>* Zusammenfassung *<br>--- | 1,10,12 | C 07 D 513/04<br>C 07 D 237/04<br>C 07 C 331/28<br>A 01 N 43/90 //<br>(C 07 D 513/04<br>C 07 D 285:00<br>C 07 D 237:00 ) |
| X | GB-A-2 163 427 (NIPPON SODA)<br>* Patentanspruch 9 * | 9 | |
| Y | * Patentansprüche 1,6 *<br>--- | 1,12 | |
| D,Y | EP-A-0 273 417 (KUMIAI CHEM. IND.)<br>* Patentansprüche 1,11 *<br>----- | 1,12 | |

RECHERCHIERTE SACHGEBIETE (Int. Cl.5)

C 07 D 513/00
C 07 D 237/00
C 07 C 331/00
A 01 N 43/00

Der vorliegende Recherchenbericht wurde für alle Patentansprüche erstellt

| Recherchenort | Abschlußdatum der Recherche | Prüfer |
|---|---|---|
| DEN HAAG | 14-06-1991 | VOYIAZOGLOU D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)